(19) Europäisches Patentamt
European Patent Office
Office européen des brevets

(11) Publication number: 0 047 516 B1

# (12) EUROPEAN PATENT SPECIFICATION

(45) Date of publication of patent specification: 04.01.84

(21) Application number: 81106970.7

(22) Date of filing: 04.09.81

(51) Int. Cl.³: C 07 C 119/14, C 07 C 121/43, C 07 D 211/88 //C07C99/00, C07C101/20, C07C103/183

## (54) Propylamine derivative and process of manufacturing the same.

(30) Priority: 04.09.80 JP 121747/80

(43) Date of publication of application: 17.03.82 Bulletin 82/11

(45) Publication of the grant of the patent: 04.01.84 Bulletin 84/1

(84) Designated Contracting States: DE FR GB IT

(56) References cited:
GB - A - 2 018 755
GB - A - 2 021 559
US - A - 4 130 717

(73) Proprietor: TORAY INDUSTRIES, INC.
2, Nihonbashi-Muromachi 2-chome Chuo-ku
Tokyo 103 (JP)

(72) Inventor: Sato, Haruyo
2-28, Uchide-cho Nakagawa-ku
Nagoya-shi Aichi-ken (JP)
Inventor: Imamura, Shinzo
2-1, Toray Matsuzono-shataku 2-19, Matsuzono-cho
Mizuho-ku Nagoya-shi Aichi-ken (JP)

(74) Representative: Wey, Hans-Heinrich, Dipl.-Ing. et al,
Patentanwälte Müller-Börner Wey & Körner
Widenmayerstrasse 49
D-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 047 516 B1

Courier Press, Leamington Spa, England.

Propylamine derivative and process of manufacturing the same

*Background of the invention*

(1) Field of the invention

The present invention relates to a propylamine derivative and a process of manufacturing the same. The propylamine derivative obtained by the present invention may readily lead to $\alpha$-amino acids having functional groups such as glutamine, proline, ornithine, and glutamic acid, by further effecting hydrolysis, and hydrogenation.

(2) Description of the prior art.

Conventionally, there have been known various processes for producing $\alpha$-amino acids, of which, for example, the following methods (1) to (3) have recently been proposed as improved processes for effecting reactions under mild conditions.

(1) 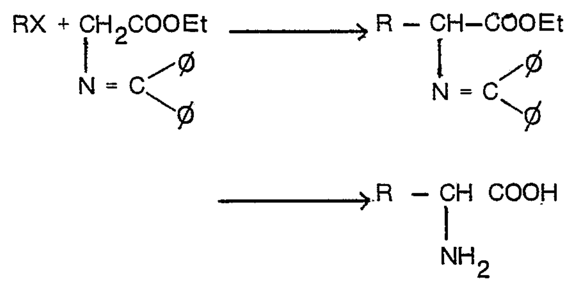

R = alkyl group,

aralkyl group

X = I, Br

[Tetrahedron Lett. 2641 (1978)]

(2) 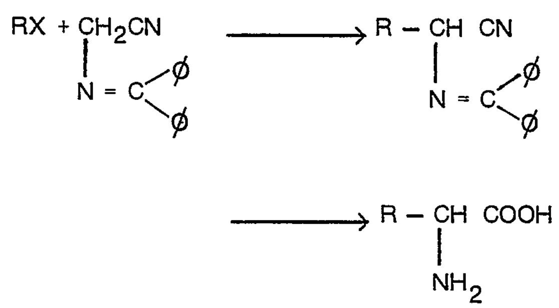

R = alkyl group,

aralkyl group

X = Cl, Br, $CH_3OSO_3$

[Tetrahedron Lett. 4625 (1978)]

(3) 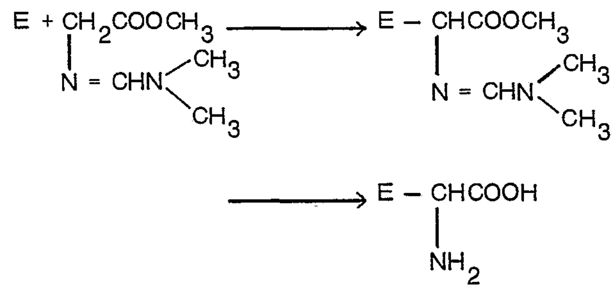

E (electrophile) ; cinnamyl bromide

dimethyl p-chlorobenzalmalonate

nitrostyrene

[J. Org. Chem. *42* 2640 (1977)]

However, the known methods as descibed above still have some problems to be solved with respect to the points as follows.

(a) In those methods, the Schiff bases are produced under mild conditions at ordinary temperature and atmospheric pressure, but the required reaction times are rather long.

(b) In each case of the above methods (1), (2), and (3), since an alkylhalide is employed as the material for producing said Schiff bases, it is necessary to further add a functional group on the alkyl radical of the Schiff bases, to get an intermediate for producing an amino acid having three functional groups, for example, glutamine, proline, ornithine, and glutamic acid.

However, owing the fact that the Schiff bases are very sensitive to the chemical reaction, it is difficult to effect a specific reaction for introduction of a new functional group, and thus, normally, several kinds of products are undesirably obtained simultaneously.

(c) In method (3), amidine represented by

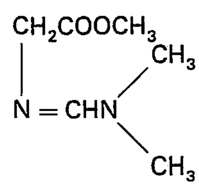

and employed as a starting material is an expensive reagent in itself, is difficult to synthesize, and therefore is unsuitable to be used as a starting material industrially.

(d) The yields of 70 to 80% are rather low and are considered to be insufficient for an industrial production.

Therefore, it has been strongly desired to provide a process of synthesizing an amino acid intermediate which is more economical with a higher yield and a better suitability for general purposes than the conventional techniques as described above.

GB—A 2 021 559 relates to the benzylidene derivatives corresponding to the following formula:

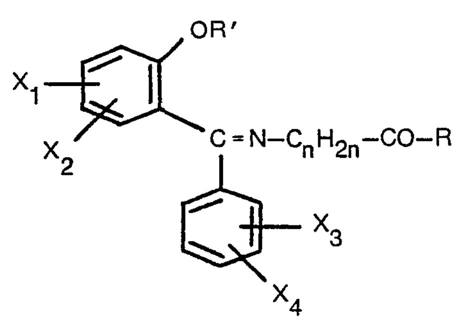

and GB—A 2 018 755 relates to the benzylidene esters corresponding to the following formula:

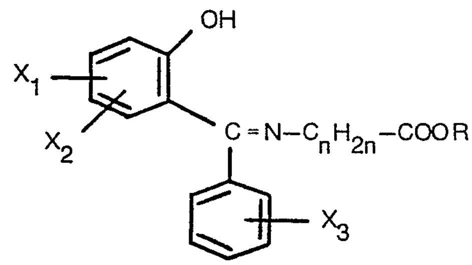

These benzylidene derivatives and benzylidene esters are different from the propylamine derivatives according to the present invention.

The proplamine derivatives obtained in the present invention (hereinafter referred to "the present compound"), which are represented by the above formula (I), may readily lead to $\alpha$-amino acid having functional groups such as glutamine, proline, ornithine, and glutamic acid, by converting $R^2$ radical to COOH radical and by converting

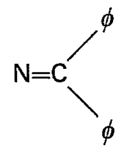

radical to $NH_2$ radical by hydrolysis.

Therefore, in the present compound, $R^2$ is the important radical which can be COOH radical of $\alpha$-amino acid.

While both the benzylidene derivatives and the benzylidene esters have not the radical which can be COOH radical of $\alpha$-amino acid. Therefore, the benzylidene derivatives cannot be led to $\alpha$-amino acid.

US—A 4 130 717 relates to the malonic acid ester of the following formula:

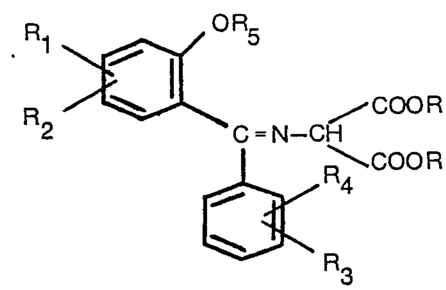

This malonic acid ester is led to the isoindole derivatives by heating, but cannot be led to $\alpha$-amino acid such as glutamine, proline, ornithine, and glutamic acid, which are useful $\alpha$-amino acids.

*Object of the invention*

Accordingly, a primary object of the present invention is to provide intermediates which are chemically stable and can be converted into several amino acids having functional groups.

A secondary object of the present invention is to provide processes of manufacturing the intermediates as described above with the employment of a material readily available, under mild conditions in good yields.

A third object of the present invention is to provide processes of manufacturing the intermediates for the production of amino acids wherein benzophenone recovered as a by-product during manufacture of amino acids from the above intermediates is recycled.

*Brief description of the invention*

These and other objects of the present invention will be accomplished by obtaining propylamine derivatives represented by the general formula (I),

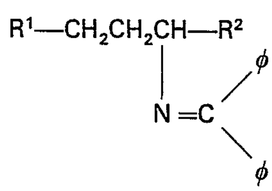

(I)

wherein $R^1$ and $R^2$ are identical or different radicals selected from the group consisting of methoxycarbonyl, ethoxycarbonyl, propoxycarbonyl, butoxycarbonyl, cyano, and carbamoyl, or $R^1$ and $R^2$ form in combination a cyclic imide, and $\phi$ is a phenyl radical.

Here, the above derivatives of the formula (I),

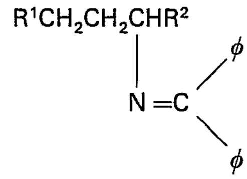 (I)

(wherein $R^1$ is a radical selected from the group consisting of methoxycarbonyl, ethoxycarbonyl, propoxycarbonyl, butoxycarbonyl, cyano and carbamoyl, and $R^2$ is a radical selected from the group consisting of methoxycarbonyl, ethoxycarbonyl, propoxycarbonyl, butoxycarbonyl and cyano, or $R^1$ and $R^2$ form in combination a cyclic imide, and $\phi$ is a phenyl radical), can be obtained by subjecting olefins represented by the general formula (II),

$$R^1 - CH = CH_2 \quad \text{(II)}$$

(wherein $R^1$ is a radical selected from the group consisting of methoxycarbonyl, ethoxycarbonyl, propoxycarbonyl, butoxycarbonyl, cyano, and carbamoyl) and Schiff bases represented by the general formula (III),

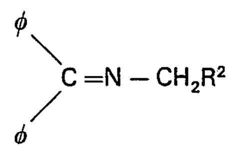 (III)

(wherein $R^2$ is a radical selected from the group consisting methoxycarbonyl, ethoxycarbonyl, propoxycarbonyl, butoxycarbonyl, and cyano, and $\phi$ is a phenyl radical), to reaction at a reaction temperature of from −80°C to 80°C, with or without use of a catalyst in the presence of bases in a monophase or two-phase solvent system.

Alternatively, the above derivatives of the formula (I),

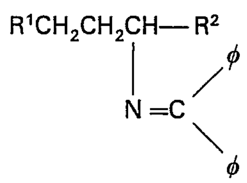 (I)

(wherein $R^1$ and $R^2$ are identical or different radicals selected from the group consisting of cyano, and carbamoyl, with at least one of the radicals for $R^1$ and $R^2$ being carbamoyl, and $\phi$ is a phenyl radical), can be obtained by subjecting the compound of the following general formula (I),

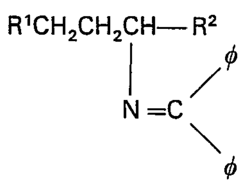 (I)

(wherein $R^1$ is a radical selected from the group consisting of methoxycarbonyl, ethoxycarbonyl, propoxycarbonyl, butoxycarbonyl, cyano, carbamoyl, and $R^2$ is a radical selected from the group consisting of methoxycarbonyl, ethoxycarbonyl, propoxycarbonyl, butoxycarbonyl and cyano, or $R^1$ and $R^2$ form in combination a cyclic imide, and $\phi$ is a phenyl radical), to reaction with ammonia at a temperature of from −20°C to 80°C under atmospheric pressure or under increased or reduced pressure in the presence of a solvent.

*Detailed description of the invention*

Hereinbelow, the processes of the present invention will be first described and, then, the novel compounds prepared by the process will be described.

In the first, olefins for the starting material represented by the formula (II) are prepared.

$$R^1 - CH = CH_2 \qquad (II)$$

wherein $R^1$ is a radical selected from the group consisting of methoxycarbonyl, ethoxycarbonyl, propoxycarbonyl, butoxycarbonyl, cyano, and carbamoyl. Olefins as described above are methylacrylate, ethylacrylate, propylacrylate, butylacrylate, acrylonitrile or acrylamide.

Meanwhile, Schiff bases represented by the general formula (III) are prepared as the other starting material.

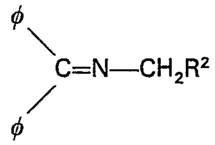

(III)

wherein $R^2$ is a radical selected from the group consisting of methoxycarbonyl, ethoxycarbonyl, propoxycarbonyl, butoxycarbonyl, and cyano, and $\phi$ is a phenyl radical.

The Schiff bases of formula (III) usable for the present invention may be prepared by two processes.

One is a process wherein a glycine ester obtained by reacting glycine with an alcohol of 1 to 4 carbon atoms is subjected to a dehydration reaction with benzophenone [Tetrahedron Lett., No. 30, 2641 (1978)]. The other is a process wherein glycinonitrile is reacted with benzophenone [Tetrahedron Lett., No. 30, 4625 (1978)]. N-(diphenylmethylene) glycine alkyl esters are obtained by the former process and N-(diphenylmethylene)glycinonitrile is obtained by the latter process. As the N-(diphenylmethylene) glycine alkyl esters, there may be mentioned methyl N-(diphenylmethylene) glycinate, ethyl N-(diphenylmethylene) glycinate, propyl N-(diphenylmethylene) glycinate and butyl N-(diphenylmethylene) glycinate.

Olefins and Schiff bases thus obtained are reacted together. The reaction may be effected by two processes. One is a process wherein the reaction is effected in a homogeneous solvent, i.e. a monophase solvent system, the other is a process wherein the reaction is effected in a two-phase solvent system consisting of an aqueous phase and an organic phase.

The reaction is carried out in the solvent, in the presence of a base, with or without the use of a catalyst, and at a temperature of from −80°C to 80°C.

Although the catalyst is not required for the reaction in the monophase solvent system it is preferable to employ a catalyst for effecting the reaction by the two phase solvent system.

As the catalyst, there may be employed halides, hydrogensulfates or hydroxides of tetraalkylammoniums or benzyltrialkylammoniums, having 1 to 16 carbon atoms in each alkyl group, preferably quaternary ammonium salts such as tetrabutylammonium hydrogensulfate, benzyltrimethylammonium chloride, benzyltrimethylammonium hydroxide, cetyltrimethylammonium bromide, dodecyltrimethylammonium chloride and tetraethylammonium hydroxide, more preferably tetrabutylammonium hydrogensulfate, benzyltrimethyl ammonium hydroxide and tetraethylammonium hydroxide. The catalyst may be employed in an amount of from 0.0001 to 1 equivalent, preferably 0.001 to 1 equivalent, based on the Schiff base. If the catalyst is employed in an amount less than 0.0001 equivalent, both the yield of the final product and the reaction rate are low. If the amount of the catalyst is larger than 1 equivalent, it is difficult to isolate the final product from the reaction mixture.

The reaction temperature should be from −80°C to 80°C, preferably −80°C to 40°C. Where the temperature is too low, the reaction proceeds too slowly. When the temperature is too high, side reactions may undesirably occur and, further, in the case of the reaction in the two-phase solvent system, hydrolysis may undesirably occur.

The base may be employed in an amount of from 0.001 to 10 equivalents, preferably 0.001 to 5 equivalents, based on the Schiff base.

In the case of the reaction in the monophase solvent system, there may be employed, as the solvent, aliphatic alcohols such as methanol, ethanol, propanol and butanol, halogenated hydrocarbons such as dichloromethane, chloroform, trichloroethylene and trichloroethane, aromatic hydrocarbons such as benzene, toluene and xylene, ethers such as diethyl ether, tetrahydrofuran and dioxane, dimethylformamide, and dimethyl sulfoxide. When these solvents are employed in the reaction system, it is preferable to employ a base selected from alkali metals and their hydrides, hydroxides, amides and alcoholates.

In the case of the reaction in the two-phase solvent system, there may be employed a mixture of water with at least one organic solvent selected from the above-mentioned halogenated hydrocarbons, ethers, aromatic hydrocarbons, dimethylformamide and dimethyl sulfoxide. In such a case, there may preferably be employed, as the base, hydroxides, carbonates and hydrogencarbonates of alkali metals such as sodium hydroxide, sodium carbonate and sodium hydrogencarbonate. However, where an alkali metal hydroxide is used, chloroform and trichloroethylene should not be employed in

the solvent since explosive compounds, such as carbene and dichloroacetylene, may probably be formed in the system.

In both the above-mentioned reactions, the starting materials may be employed in an approximately stoichiometric amount, preferably 0.8 to 1.2 mol equivalent of the olefin should be employed based on the Schiff base.

The reaction may be carried out under atmospheric pressure or under increased or reduced pressure, preferably under atmospheric pressure. The reaction may be effected batch-wise, continuously or semi-continuously. In the case of the batch-wise reaction, the reaction may usually be completed in from 5 to 120 minutes.

Where the reaction is effected batch-wise, it is preferable that the olefin is added to a mixture of the Schiff base, the base, the solvent and the catalyst (if any) with stirring.

After the completion of the reaction, upon separation of the product by the known method, propylamine derivatives represented by the following formula (I) are obtained.

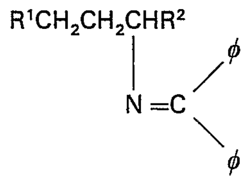

(I)

(wherein $R^1$ is a radical selected from the group consisting of methoxycarbonyl, ethoxycarbonyl, propoxycarbonyl, butoxycarbonyl, cyano and carbamoyl, and $R^2$ is a radical selected from the group consisting of methoxycarbonyl, ethoxycarbonyl, propoxycarbonyl, butoxycarbonyl, cyano, or $R^1$ and $R^2$ form in combination a cyclic imide, and $\phi$ is a phenyl radical).

After the completion of the reaction, the product of formula I, as hereinbefore defined, may be isolated in the usual way. For example, where the two-phase solvent system is employed, the solvent is removed from the organic phase which is separated from the aqueous phase after the completion of the reaction, and the residue is passed through a column packed with silica gel. As the developing solvent, cyclohexane or a mixture of cyclohexane and ethyl acetate may suitably be used. After the removal of the solvent from the eluate under reduced pressure, pure propylamine derivatives are obtained.

In the case where the desired compound is isolated from the reaction mixture obtained in the monophase solvent system, since there exists in the reaction mixture a compound which should not be brought into contact with water, such as sodium, potassium or sodium alcoholate, such a compound should first be decomposed carefully with a little water. The organic phase is concentrated, and then the product is isolated as mentioned above in a similar manner as in the two-phase solvent system.

The propylamine derivative (referred to as a cyclic imide propylamine derivative) represented by the following general formula (I),

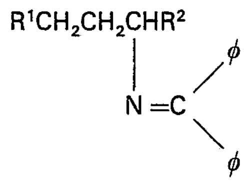

(I)

(wherein $R^1$ and $R^2$ form in combination a cyclic imide), is obtained by cyclization reaction of a propylamine derivative (referred to as an amideester propylamine derivative) which is represented by the following formula (I),

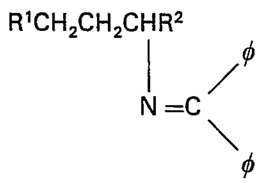

(I)

(wherein the meanings of $R^1$ and $R^2$ are as those in formulas (II) and (III) below), by reaction of olefins represented by the following formula (II),

$$R^1CH = CH_2 \qquad (II)$$

(wherein $R^1$ is a carbamoyl group), with Schiff bases represented by the following formula (III),

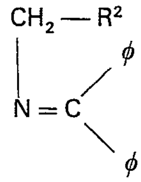

(III)

(wherein $R^2$ is a radical selected from the group consisting of methoxycarbonyl, ethoxycarbonyl, propoxycarbonyl, and butoxycarbonyl, and $\phi$ is a phenyl radical).

The cyclization reaction as described above is very fast, but under basic conditions the cyclic imide propylamine derivative thus formed is hydrolyzed and ring opening products are obtained.

In the production of the cyclic imide propylamine derivative as described above it is desirable to effect the reaction in the monophase solvent system of organic solvent substantially containing no water in order to prevent the ring opening reaction.

Meanwhile, in order to obtain the amido ester propylamine derivative at a high yield, it is preferable to adopt mild conditions employing, for example, a weak base such as sodium carbonate or the like.

Subsequently, the propylamine derivatives obtained by the reaction between olefins and Schiff bases and represented by the general formula (I),

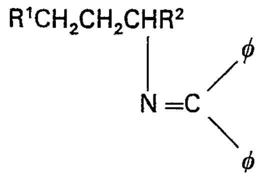

(I)

(wherein $R^1$ is a radical selected from the group consisting of methoxycarbonyl, ethoxycarbonyl, propoxycarbonyl, butoxycarbonyl, cyano and carbamoyl, and $R^2$ is a radical selected from the group consisting of methoxycarbonyl, ethoxycarbonyl, propoxycarbonyl, butoxycarbonyl and cyano, or $R^1$ and $R^2$ form in combination a cyclic imide, with at least one of $R^1$ and $R^2$ being a radical selected from the group consisting of methoxycarbonyl, ethoxycarbonyl, propoxycarbonyl, butoxycarbonyl, and $R^1$ and $R^2$ form in combination a cyclic imide and $\phi$ is a phenyl radical), are subjected to reaction with ammonia.

The ammonia to be employed in the reaction may be in any forms such as gas, liquid, or a solution, and the like.

The amount of ammonia to be employed for the reaction may be rather excessive with respect to the starting material, but in the normally such amount is 1 to 10 equivalents based on the starting material.

In the case where the ammonia is employed in a form of gas, the ammonia gas may contain any other gases which will not obstruct the reaction. If the ammonia is used in the form of a solution, 5 to 28 wt% aqueous solutions are favourably employed. The reaction temperature should be in the range of from −20°C to 80°C, and more preferably, from 0°C to 40°C. If the temperature is excessively low, the reaction proceeds too slowly, whole on the contrary, if it is too high, undesirable side reactions may take place.

Although the reaction may be effected under atmospheric pressures or under increased or reduced pressure, it should preferably be effected under atmospheric pressure.

For the solvents, there may be employed water; aliphatic alcohols such as methanol, ethanol, propanol, butanol, halogenated hydrocarbons such as dichloromethane, chloroform, trichloroethylene; aromatic hydrocarbons such as benzene, toluene, xylene; ethers such as diethyl ether, tetrahydrofuran, dioxane; and dimethylformamide, and dimethyl sulfoxide.

The amount of the solvent to be used may be set as desired, but normally, be 2 to 20 times the weight of the starting material.

The reaction can normally be effected without employment of any catalysts.

Meanwhile, the reaction may be carried out by the batch-wise, continuously or semi-continuously. In the case where the reaction is effected by the batch-wise it should normally be completed in from 0.5 to 5 hours.

Moreover, in the case of the batch-wise reaction it is preferable to gradually add ammonia into the starting material reaction mixture under stirring.

After the completion of the reaction, upon separation of the resulting product, for example, by the known method as described earlier, the propylamine derivatives as represented by the following general formula (I) are obtained.

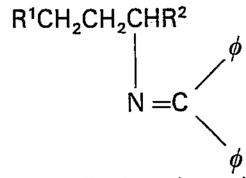

(I)

(wherein $R^1$ and $R^2$ are identical or different radicals selected from the group consisting of cyano, and carbamoyl, with at least one of $R^1$ and $R^2$ being carbamoyl, and $\phi$ is a phenyl radical.).

The crude product thus obtained is refined by the method as described earlier.

Alternatively, the cyclic imide propylamine derivative may be obtained by subjecting the propylamine derivatives (referred to as an ester ester propylamine derivatives hereinbelow) represented by the following formula (I),

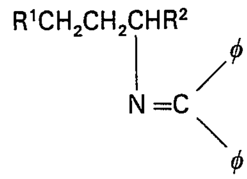

(I)

(wherein $R^1$ and $R^2$ are identical or different radicals selected from the group consisting of methoxycarbonyl, ethoxycarbonyl, propoxycarbonyl, and butoxycarbonyl, and $\phi$ is a phenyl radical), to reaction with ammonia in the similar manner as described earlier.

More specifically, in the case where the ester ester propylamine derivatives are reacted with ammonia, the propylamine derivative represented by the following formula (I),

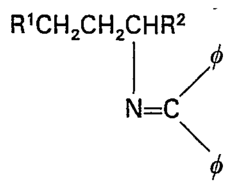

(I)

(wherein $R^1$ and $R^2$ are carbamoyl radical). is obtained, after passing through the cyclic imide propylamine derivative as described earlier.

For manufacturing the cyclic imide propylamine derivative with a favourable yield, it is preferable to prevent the diester from being directly formed into diamide by controlling the mole ratio of ammonia employed in the reaction to the range of from 0.8 to 1.1, and simultaneously to effect the reaction in an organic solvent not containing water so that the cyclic imide propylamine derivative thus formed is not subjected to the ring opening reaction by hydrolysis, and further, to carry out the reaction at as low a temperature as possible.

In connection with the above, the forms of ammonia to be employed in the reaction, reacting temperatures, reacting pressures, kinds of solvents, amounts thereof, reaction systems, reaction times, isolating methods, refining methods, etc. may be the same as in the case of the reaction between the propylamine derivative and ammonia described earlier.

According to the processes of the present invention as explained above, the following specific effects can be attained:

(1) — Desired products can be obtained in a high yield.

(2) — These processes can generally be carried out under a mild condition and, thus, are industrially advantageous.

(3) — These processes can be carried out by a simple apparatus and operation.

(4) — The Schiff bases to be used as a starting material in the present process are stable in an aqueous alkali solution and can be purified by distillation or recrystallization.

The compounds according to the present invention as described in the foregoing are novel propylamine derivatives represented by the following formula (I),

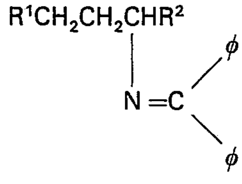

(I)

(wherein $R^1$ and $R^2$ are identical or different radicals selected from the group consisting of methoxycarbonyl, ethoxycarbonyl, propoxycarbonyl, butoxycarbonyl, cyano, and carbamoyl, or $R^1$ and $R^2$ form in

combination a cyclic imide, and $\phi$ is a phenyl radical).

The compounds according to the present invention are characterized in that (1) as shown in Table 1, they are respectively intermediates of important amino acids and capable of being induced into several of amino acids, and (2) employ benzophenone which is stable as a protecting agent of amino groups and capable of being used for recycling, and (3) are intermediates of $\alpha$-amino acids having functional groups.

Accordingly, the compounds of the present invention have advantages as follows.

(1) The novel compounds of the present invention can easily be converted into $\alpha$-amino acids having functional groups, for example, glutamine, ornithine, proline, glutamic acid, tryptophane, etc., by the necessary chemical treatments and successive hydrolysis in the presence of an acid and then of a base, or in the presence of a base and then of an acid.

As has also been stated previously, it is extremely difficult and also ineffecient to introduce functional groups into the intermediates of amino acids, after the formation of the main chain. While, introduction of the functional groups at early steps has a possibility of giving rise to side reactions, with consequent reduction of the yield on the whole.

According to the present invention, the problems as described above have simultaneously been solved, and it has been made possible to produce $\alpha$-amino acids in a high yield.

(2) During the hydrolysis of the compounds of formula (I), benzophenone is formed as a by-product. Since benzophenone has no active hydrogen in its molecule, it is stable both thermally and chemically as compared with benzaldehyde, acetophenone, etc., and therefore, may be recycled and reused as a starting material for the production of the Schiff bases usable in the present invention. This is one of the specific features of the present invention.

(3) Furthermore, since the compounds according to the present invention employ benzophenone as the protecting agent of amino groups, hydrolysis and other side reactions may be advantageously suppressed, and thus, reaction points aimed at can be specified.

(4) Consequently, it is possible to manufacture the compounds of the present invention in a very high yield.

TABLE 1

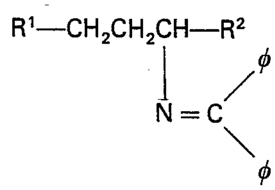

| $R^1$ | $R^2$ | Derivable amino acids |
|---|---|---|
| Ester | Ester<br>Nitrile<br>Amide | Glutamine, Glutamic acid |
| Nitrile | Ester<br>Nitrile<br>Amide | Ornithine, Proline,<br>Tryptophane, Glutamine<br>Glutamic acid,<br>Arginine, Citrulline |
| Amide | Ester<br>Nitrile<br>Amide | Glutamine, Glutamic acid |
| Cyclic imide | | Glutamine, Glutamic acid |

Example 1

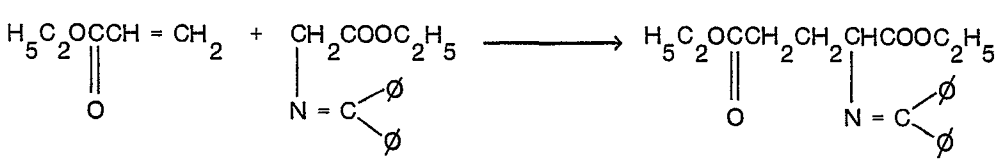

A mixture of 5.3 g (0.02 mol) of ethyl N-(diphenylmethylene)glycinate, 2.0 g (0.02 mol) of ethyl

acrylate, 0.7 g (0.002 mol) of tetrabutylammonium hydrogensulfate, 50 ml of dichloromethane, and 5 ml of 4 N aqueous sodium hydroxide solution were introduced into an Erlenmyer flask and stirred at room temperature for 1 hour.

Then, the reaction mixture was subjected to liquid separation, the dichloromethane layer was washed with water and dried.

The solvent was distilled off under reduced pressure. Thus, 6.6 g (89.2%) of crude N-(diphenylmethylene)-1,3-bis(ethoxycarbonyl)propylamine was obtained.

The crude product was placed on a silica gel column and eluted with 1:9 (v/v) ethylacetate-cyclohexane. Fractions containing product were combined and evaporated, affording 5.7 g of N-(diphenylmethylene)-1,3-bis(ethoxycarbonyl)propylamine as an oil. The analytical values of this compound are given in Table 2.

Example 2—5

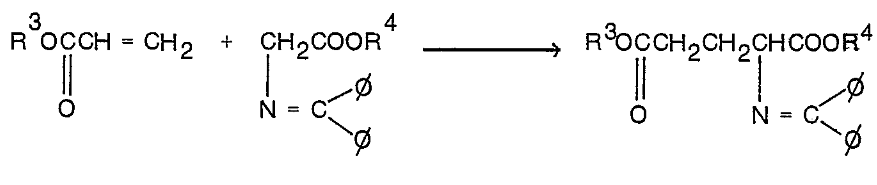

N-(diphenylmethylene)-1,3-bis(alkoxycarbonyl)propylamines were prepared by reaction of alkyl acrylates with alkyl-N-(diphenylmethylene)glycinates in the way described in Example 1 on using equivalent amount of the corresponding starting materials. These examples are summarized in the following Table 3.

TABLE 3

| Example No. | $R^3$ | $R^4$ | Yield (%) |
|---|---|---|---|
| 2 | $CH_3$ | $C_2H_5$ | 88.7 |
| 3 | $C_4H_9$ | $C_2H_5$ | 90.1 |
| 4 | $C_4H_9$ | $C_4H_9$ | 90.6 |
| 5 | $C_4H_9$ | $CH_3$ | 87.4 |

The analytical values of these compounds are given in Table 2.

Example 6

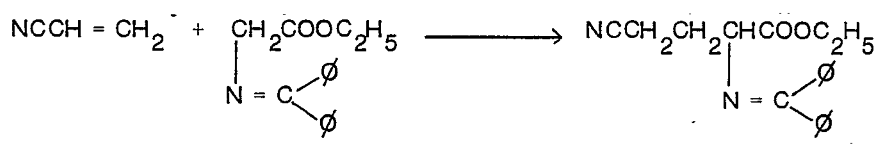

A mixture of 2.7 g (0.01 mol) of ethyl N-(diphenylmethylene)glycinate, 0.5 g (0.01 mol) of acrylonitrile, 0.3 g (0.002 mol) of tetrabutylammonium hydrogensulfate, 20 ml of dichloromethane, and 1.1 g of 35 wt% aqueous sodium hydroxide solution were introduced into an Erlenmyer flask and stirred at room temperature for half an hour.

Then, the reaction mixture was subjected to liquid separation, the dichloromethane layer was washed with water and dried.

The solvent was distilled off under a reduced pressure.

Thus, 2.9 g (90.5%) of crude N-(diphenylmethylene)-3-cyano-1-ethoxycarbonylpropylamine was obtained.

The crude product was placed on a silica gel column and eluted with 1:9 (v/v) ethylacetate-cyclohexane. Fractions containing product were combined and evaporated, affording 2.7 g of N-(diphenylmethylene)-3-cyano-1-ethoxycarbonylpropylamine as a pale yellow oil.

The analytical values of these compounds are given in Table 2.

Example 7

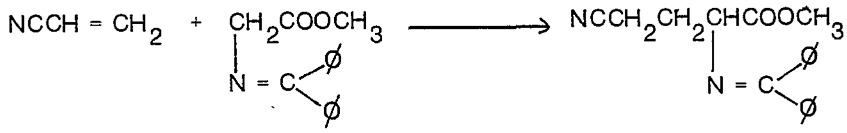

N-(diphenylmethylene)-3-cyano-1-methoxycarbonylpropylamine was prepared by reaction of acrylonitrile with methyl N-(diphenylmethylene)glycinate in a similar manner as Example 6.

The analytical values and the yield of this compound are given in Table 2.

Example 8

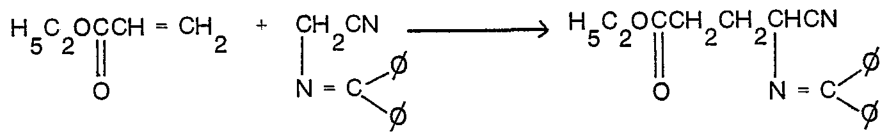

A mixture of 4.4 g (0.02 mol) of N-(diphenylmethylene)glycinonitrile, 2.0 g (0.02 mol) of ethyl acrylate, 0.3 g (0.001 mol) of tetrabutylammonium hydrogensulfate, 50 ml of dichloromethane, and 3.0 g of 35 wt% aqueous sodium hydroxide solution were introduced into an Erlenmyer flask and stirred at room temperature for 1 hour.

Then, the reaction mixture was subjected to liquid separation, the dichloromethane layer was washed with water and dried.

The solvent was distilled off under a reduced pressure.

Thus, 6.1 g (95.3%) of crude N-(diphenylmethylene)-1-cyano-3-ethoxycarbonylpropylamine was obtained.

The crude product was placed on a silica gel column and eluted with 1:9 (v/v) ethylacetate-cyclohexane. Fractions containing product were combined and evaporated, affording 5.8 g of N-(diphenylmethylene)-1-cyano-3-ethoxycarbonylpropylamine as a pale yellow oil.

The analytical values and the yield of this compound are given in Table 2.

Example 9—10

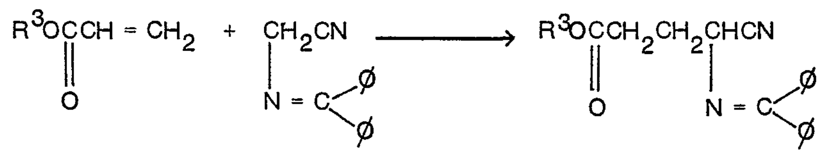

N-(diphenylmethylene)-3-alkoxycarbonyl-1-cyanopropylamines were prepared by reaction of alkyl acrylates with N-(diphenylmethylene)glycinonitrile in a similar manner as Example 8.

These examples were summarized in the following Table 4.

TABLE 4

| Example No. | $R^3$ | Yield (%) |
|---|---|---|
| 9 | $CH_3$ | 91.4 |
| 10 | $C_4H_9$ | 90.9 |

The analyatical value of these compounds are given in Table 2.

Example 11

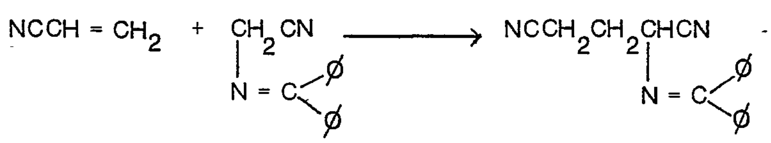

A mixture of 4.4 g (0.02 mol) of N-(diphenylmethylene)glycinonitrile, 1.1 g (0.02 mol) of acrylonitrile, 0.2 g (0.001 mol) of benzyltrimethylammonium chloride, 50 ml of dichloromethane and 3.0 g of 35 wt% aqueous sodium hydroxide solution were introduced into an Erlenmyer flask and stirred at 5 to 10°C for 1 hour.

Then, the reaction mixture was subjected to liquid separation, the dichloromethane layer was washed with water and dried.

The solvent was distilled off under a reduced pressure.

Thus, 4.8 g (87.9%) of crude N-(diphenylmethylene)-1,3-dicyanopropylamine was obtained.

The crude product was placed on a silica gel column and eluted with 1:9 (v/v) ethylacetate-cyclohexane. Fractions containing product were combined and evaporated, affording 4.4 g of N-(diphenylmethylene)-1,3-dicyanopropylamine as a pale yellow solid.

The product was recrystallized from a mixture of ethyl acetate and cyclohexane, and 3.9 g of pure N-(diphenylmethylene)-1,3-dicyanopropylamine was obtained, which melted at 83 to 84°C.

The analytical values of this compound are given in Table 2.

## Example 12

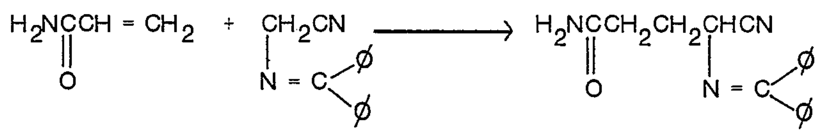

A mixture of 4.4 g (0.02 mol) of N-(diphenylmethylene)glycinonitrile, 1.4 g (0.02 mol) of acrylamide, 0.3 g (0.001 mol) of tetrabutylammonium hydrogensulfate, 50 ml of dichloromethane, and 5.0 g of 35 wt% aqueous sodium hydroxide solution were introduced into an Erlenmyer flask and stirred at room temperature for 1 hour.

Then, the reaction mixture was subjected to liquid separation, the dichloromethane layer was washed with water and dried.

The solvent was distilled off under a reduced pressure. Thus, 5.3 g (91.4%) of crude N-(diphenylmethylene)-3-carbamoyl-1-cyanopropylamine was obtained.

The crude product was placed on a silica gel column and eluted with 1:9 (v/v) ethylacetate-cyclohexane. Fractions containing product were combined and evaporated, affording 5.1 g of propylamine derivative as a white solid.

The product was recrystallized from a mixture of ethyl acetate and cyclohexane, and 4.5 g of pure N-(diphenylmethylene)-3-carbamoyl-1-cyanopropylamine was obtained, which melted at 131 to 132°C.

The analytical values of this compound are given in Table 2.

## Example 13

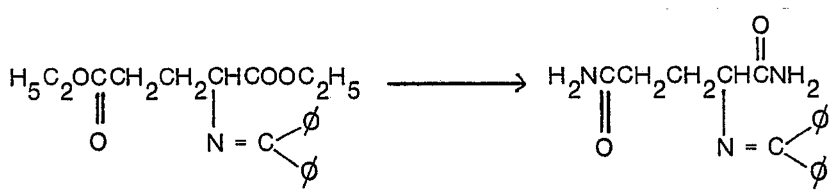

A mixture of 3.7 g (0.01 mol) of N-(diphenylmethylene)-1,3-bis(ethoxycarbonyl)propylamine, 10 ml of methanol and 20 ml of 28 wt% aqueous ammonia solution were introduced into an Erlenmyer flask and stirred at room temperature for 10 hours.

Then, the reaction mixture was distilled off under a reduced pressure.

Thus, 2.4 g (77.4%) of crude N-(diphenylmethylene-1,3-bis(carbamoyl)propylamine was obtained.

The crude product was recrystallized from a mixture of ethyl acetate and cyclohexane, and yielded 2.1 g of pure N-(diphenylmethylene)-1,3-bis(carbamoyl)propylamine, which melted at 217 to 219°C. The analytical values of this compound are given in Table 2.

## Example 14

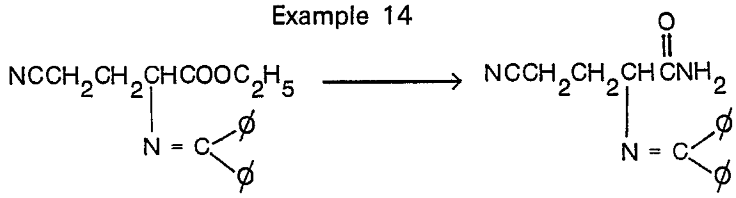

A mixture of 3.2 g (0.01 mol) of N-(diphenylmethylene)-3-cyano-1-ethoxycarbonylpropylamine, 10 ml of methanol and 20 ml of 28 wt% aqueous ammonia solution were introduced into an Erlenmyer flask and stirred at room temperature for 10 hours.

Then, the reaction mixture was distilled off under a reduced pressure.

Thus, 2.6 g (88.6%) of crude N-(diphenylmethylene)-1-carbamoyl-3-cyanopropylamine was obtained.

The crude products was recrystallized from a mixture of ethyl acetate and cyclohexane, and yielded 2.3 g of pure N-(diphenylmethylene)-1-carbamoyl-3-cyanopropylamine, which melted at 141 to 142°C. The analytical values of this compound are given in Table 2.

Example 15

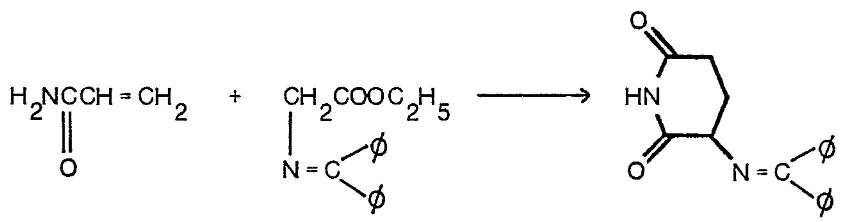

A mixture of 5.4 g (0.02 mol) of ethyl N-(diphenylmethylene)glycinate, 1.4 g (0.02 mol) of acrylamide, 50 ml of anhydrous ethanol and 1.4 g (0.002 mol) of sodium ethylate were introduced into an Erlenmyer flask and stirred at room temperature for 1 hour.

Then, the reaction mixture was distilled off under a rereduced pressure.

The residue was dissolved in 20 ml of chloroform and cooled to 0°C in an ice-salt bath. After 20 ml of water was added slowly to the solution, it was stirred at 0°C for 10 minutes.

Then, the mixture was subjected to liquid separation and the water layer was extracted with 20 ml of chloroform. The chloroform solution was dried and distilled off under a reduced pressure.

Thus, 5.3 g (90.8%) of crude N-(diphenylmethylene)-1,3-dicarboxyiminopropylamine was obtained.

The crude product was recrystallized from a mixture of ethyl acetate and cyclohexane, and yielded 5.0 g of pure N-(diphenylmethylene)-1,3-dicarboxyiminopropylamine, which melted at 128 to 129°C. The analytical values of this compound are given in Table 2.

Example 16

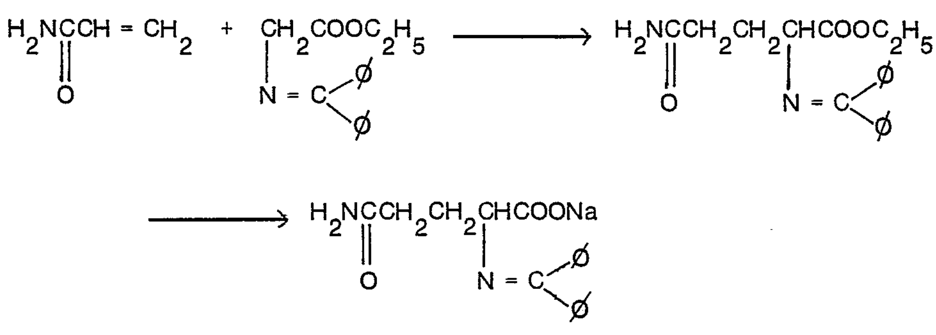

A mixture of 5.3 g (0.02 mol) of ethyl N-(diphenylmethylene)glycinate, 1.4 g (0.02 mol) of acrylamide, 0.7 g (0.002 mol) of tetrabutylammonium hydrogensulfate, 30 ml of dichloromethane, and 5 ml of 8 N aqueous sodium hydroxide solution were introduced into an Erlenmyer flask and stirred at room temperature for 2 hours.

A clear solution was obtained momentarily but in a short time a white precipitate was appeared. The precipitate was collected on a glass filter, and washed with 10 ml of 90 vol% aqueous ethanol solution and dried.

Thus, 6.3 g (94.1%) of crude $N^{\alpha}$-(diphenylmethylene)glutamine sodium salt was obtained.

The crude product was recrystallized from methanol, and yielded 5.7 g of pure $N^{\alpha}$-(diphenylmethylene)glutamine sodium salt, which melted at 204 to 205°C.

The analytical values of this compound are given in Table 5.

TABLE 5

| | | | | | |
|---|---|---|---|---|---|
| elemental analysis | | C(%) | H(%) | N(%) | Na(%) |
| ($C_{18}H_{17}N_2O_3Na$) | calc. | 65.05 | 5.16 | 8.43 | 6.92 |
| | found | 65.24 | 5.07 | 8.41 | 6.90 |
| IR ($cm^{-1}$) | 3540, | 3430, | 3120, | 1670, | 1580, |
| (KBr tablet) | 1400 | | | | |
| $^1H$ NMR $\delta$(ppm) | 1.9—2.1 (4H), 3.5—3.8 (1H), | | | | |
| ($D_2O$) | 6.7—7.5 (10H) | | | | |
| $^{13}C$ NMR $\delta$(ppm) | 31 (1C), 33 (1C), 68 1C), | | | | |
| (DMSO—$d_6$) | 128—140 (12C), 164 (1C), 174 (2C) | | | | |

Example 17

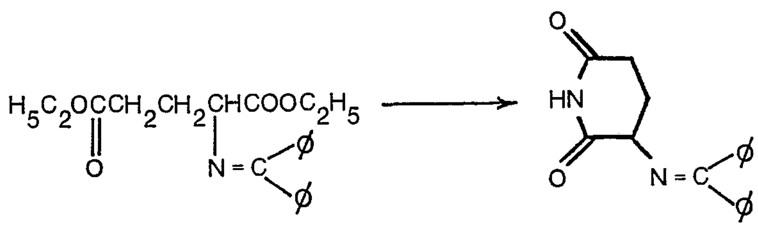

A 100 ml of three-necked flask was equipped with a mechanical stirrer, a thermometer and a dropping funnel.

The flask was charged with 3.7 g (0.01 mol) of diethyl N-(diphenylmethylene)glutamate and 30 ml of ethanol.

A solution of 0.2 g (0.012 mol) of ammonia in a 10 ml of ethanol was added over half an hour to the contents of the flask with stirring at room temperature.

The mixture was stirred for another 2 hours and then evaporated in a reduced pressure, affording crude N-(diphenylmethylene)-1,3-dicarboxyiminopropylamine.

The crude product was recrystallized from a mixture of ethyl acetate and cyclohexane, and yielded 2.5 g (86.2%) of pure N-(diphenylmethylene)-1,3-dicarboxyiminopropylamine, which melted at 128 to 129°C.

Example 18

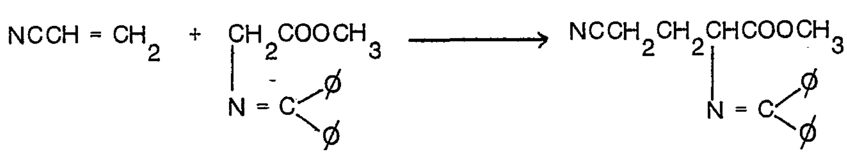

A mixture of 2.5 g (0.01 mol) of methyl N-(diphenylmethylene)glycinate, 0.5 g (0.01 mol) of acrylonitrile, 1.7 g (0.005 mol) of tetrabutylammoniun hydrogensulfate, 20 ml of dichloromethane and 5.6 g of 30 wt% aqueous potassium hydroxide solution were introduced into an Erlenmyer flask and stirred at 0 to 5°C for 1 hour.

Then the mixture was treated in a similar manner as Example 6, and obtained 2.7 g (87.3%) of N-(diphenylmethylene)-3-cyano-1-methoxycarbonylpropylamine.

Comparative Example 1

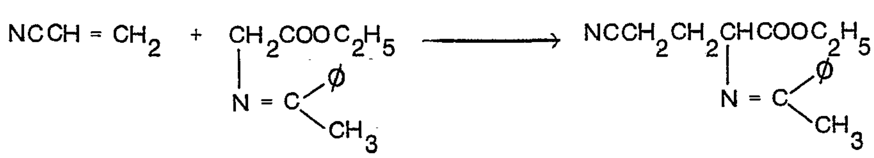

A mixture of 2.1 g (0.01 mol) of ethyl N-(methylphenylmethylene)glycinate, 0.5 g (0.01 mol) of acrylonitrile, 0.5 g (0.0025 mol) of benzyltrimethylammonium chloride, 20 ml of dichloromethane, and 2 g of 10 wt% aqueous sodium hydroxide solution were introduced into an Erlenmyer flask and stirred at room temperature for 1 hour.

The mixture was treated in a similar manner as Example 6, and obtained 1.8 g (69.7%) of N-(methylphenlmethylene)-3-cyano-1-ethoxycarbonylpropylamine.

Comparative Example 2

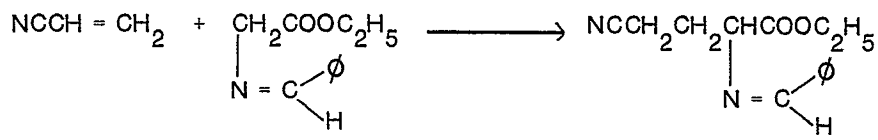

A mixture of 1.9 g (0.01 mol) of ethyl N-(monophenylmethylene)glycinate, 0.5 g (0.01 mol) of acrylonitrile, 0.5 g (0.0025 mol) of benzyltrimethylammonium chloride, 20 ml of dichloromethane and 5 g of 10 wt% aqueous potassium hydroxide solution were introduced into an Erlenmyer flask and stirred at 0 to 5°C for 1 hour.

The mixture was treated in a similar manner as Example 6, and obtained 1.2 g (49.1%) of N-(phenylmethylene)-3-cyano-1-ethoxycarbonylpropylamine.

Example 19

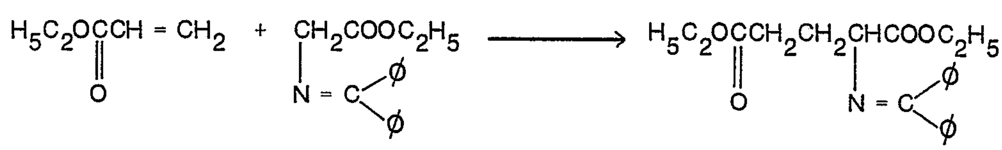

In a 100 ml of three-necked flask, fitted with a reflux condenser, a thermometer and a mechanical stirrer, were placed 2.7 g (0.01 mol) of ethyl N-(diphenylmethylene) glycinate, 0.5 g (0.01 mol) of ethyl acrylate, 50 ml of anhydrous diethylether and 0.024 g (0.001 mol) of sodium hydride and stirred at 0°C for 2 hours.

Then, 5 ml of water was added slowly to the reaction mixture and stirred at 0°C for 10 minutes. The ether layer, separated from the reaction mixture, was dried and distilled under a reduced pressure.

Thus, 3.5 g (94.6%) of crude N-(diphenylmethylene)-1,3-bis(ethoxycarbonyl)propylamine was obtained.

The crude product was treated in a similar manner as Example 1, 3.1 g of pure N-(diphenylmethylene)-1,3-bis-(ethoxycarbonyl)propylamine was obtained.

Example 20

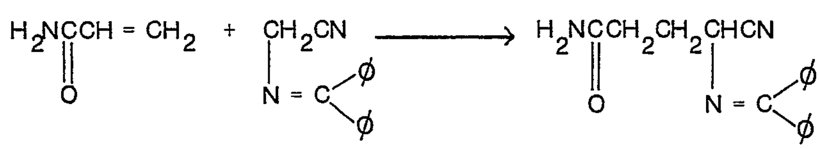

A mixture of 2.2 g (0.01 mol) of N-(diphenylmethylene)glycinonitrile, 0.7 g (0.01 mol) of acrylamide, 0.14 g (0.002 mol) of sodium ethylate and 30 ml of benzene were introduced into an Erlenmyer flask and stirred at 5 to 10°C for 1 hour.

Then, 5 ml of water was added slowly to the reaction mixture and stirred at 5 to 10°C for 10 minutes. The benzene layer, separated from the reaction mixture, was dried and evaporated. The residue was recrystallized from a mixture of ethyl acetate and cyclohexane, affording 2.6 g (89.7%) of N-(diphenylmethylene)-3-carbamoyl-1-cyanopropylamine.

Example 21

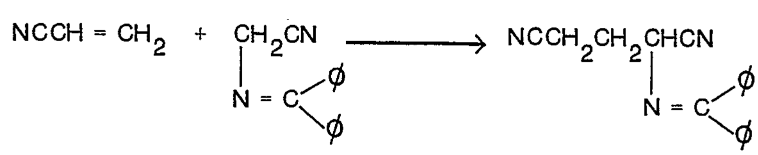

A mixture of 2.2 g (0.01 mol) of N-(diphenylmethylene)glycinonitrile, 0.5 g (0.01 mol) of acrylonitrile, 30 ml of absolute ethanol and 0.05 g (0.002 mol) of sodium metal were introduced into an Erlenmyer flask and stirred at −5 to 0°C for 1 hour.

Then, the reaction mixture was treated in a similar manner as Example 15, and obtained 2.5 g (91.6%) of N-(diphenylmethylene)-1,3-dicyanopropylamine.

Example 22

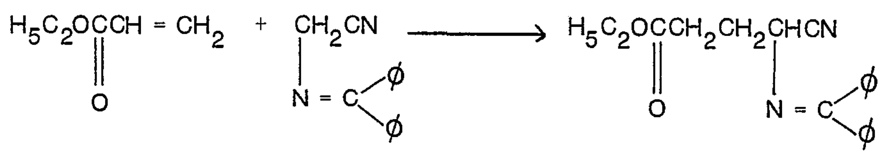

In a 200 ml four-necked flask, fitted with a reflux condenser, a thermometer, a mechanical stirrer, and a dropping funnel, were placed 5 ml (0.01 mol) of 2 N n-butyllithium hexane solution and 100 ml of freshly distilled tetrahydrofuran and stirred at 0°C, and was added 2.0 ml (0.014 mol) of dry diisopropylamine.

The colorless solution was then stirred for 30 min, at 0°C, cooled to −78°C, and treated dropwise with 2.2 g (0.01 mol) of N-(diphenylmethylene)glycinonitrile.

The resulting reaction mixture was stirred at the same temperature for 1 hour and subsequently treated with 1.0 g (0.01 mol) of ethyl acrylate. Stirring was continued at −78°C for 1 hour and at 0°C for half an hour.

Then, 5 ml of water was added slowly to a reaction mixture and stirred 0 to 5°C for 10 minutes.

The reaction mixture was treated in a similar manner as Example 15 and obtained 3.0 g (93.8%) of N-(diphenylmethylene)-1-cyano-3-ethoxycarbonylpropylamine.

Example 23

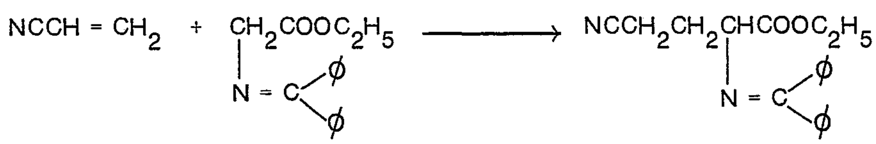

A mixture of 2.7 g (0.01 mol) of ethyl N-(diphenylmethylene)glycinate, 0.5 g (0.01 mol) of acrylonitrile, 30 ml of benzene and 3.5 g of 35 wt% aqueouss sodium hydroxide solution were introduced into an Erlenmyer flask and stirred at 50°C for 3 hours.

Then, the mixture was treated in a similar manner as Example 6, and obtained 1.7 g (53.1%) of N-(diphenylmethylene)-3-cyano-1-ethoxycarbonylpropylamine.

Example 24

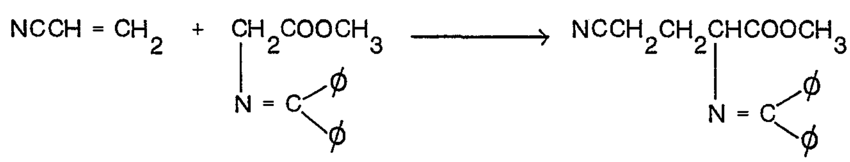

A mixture of 2.5 g (0.01 mol) of methyl N-(diphenylmethylene)glycinate, 0.5 g (0.01 mol) of acrylonitrile, 0.07 g (0.001 mol) of sodium ethylate and 20 ml of ethanol were introduced into an Erlenmyer flask and stirred at 0 to 5°C for 1 hour.

The mixture was treated in a similar manner as Example 15, and obtained 2.9 g (95.3%) of N-(diphenylmethylene)-3-cyano-1-methoxycarbonylpropylamine.

Example 25

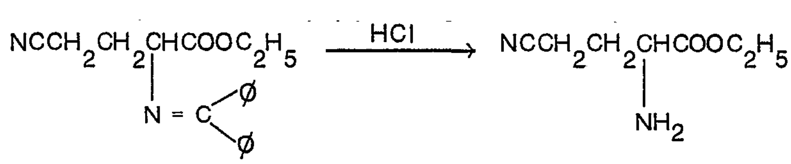


A mixture of 6.4 g (0.02 mol) of N-(diphenylmethylene)-3-cyano-1-ethoxycarbonylpropylamine, 20 ml of ethanol and 4.2 g (0.04 mol) of 35 wt% aqueous hydrochloric acid were introduced into an Erlenmyer flask and stirred at room temperature for 1 hour.

Then, the reaction mixture was distilled off under a reduced pressure. The residue was dissolved in a mixture of 30 ml of water and 30 ml of benzene.

Then, the water layer was separated, and the benzene layer was extracted with 30 ml of water. The combined water layers were neutralized with 28 wt% aqueous ammonia solution and extracted with two 50 ml portions of chloroform. The organic extracts were dried, and evaporated. The residue was distilled under reduced pressure and 3-cyano-1-ethoxy-carbonylpropylamine was collected at 103 to 106°C/0.8 mmHg.

The yield of 3-cyano-1-ethoxycarbonylpropylamine was 2.7 g (86.5%).

On the other, the benzene layer was distilled under reduced pressure, and 3.4 g (93.4%) of benzophenone was collected at 105 to 110°C/1.0 mmHg.

Further, in a 100 ml three-necked flask, fitted with a reflux condenser, a thermometer and a mechanical stirrer, were placed 2.7 g (0.015 mol) of recovered benzophenone, 1.03 g (0.01 mol) of ethyl glycinate, 30 ml of toluene, and 0.02 ml of boron trifluoride etherate. The mixture was heated for 6 hours, separating the water by azeotropic distillation.

Then, the reaction mixture was cooled to 20°C, and washed with 10 wt% aqueous sodium carbonate solution and dried. The toluene was evaporated from the organic layer and the residue was distilled under reduced pressure. Ethyl N-(diphenylmethylene)glycinate was collected at from 160 to 165°C/0.1 mmHg.

The yield of the product was 23.8 g (89.1%).

Thus, recovered benzophenone could be reused.

Example 26

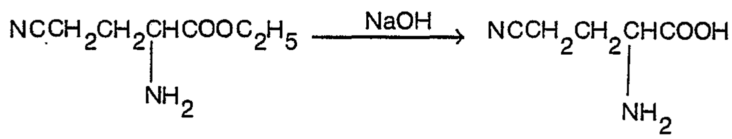


A mixture of 1.6 g (0.01 mol) of 3-cyano-1-ethoxycarbonyl-propylamine, 5 ml of ethanol and 3 g of 35 wt% aqueous sodium hydroxide solution were introduced into an Erlenmyer flask and stirred at room temperature for 1 hour.

Then, the mixture was evaporated and the resulting aqueous solution was passed through an anion-exchange resin (PA 316 made by Mitsubishi Chemical Ind. Ltd, Japan) to give 1.1 g (85.9%) of 1-carboxyl-3-cyanopropylamine.

Example 27

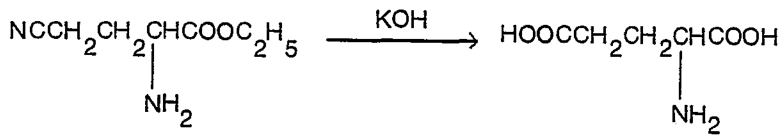


In a 100 ml of flask, fitted with a reflux condenser were placed 1.6 g (0.01 mol) of 3-cyano-1-ethoxycarbonylpropylamine and 10 ml of 20 wt% aqueous potassium hydroxide solution and heated at 80°C for 1 hour.

Then, the mixture was treated in a similar manner as Example 26, and obtained 1.2 g (81.6%) of glutamic acid.

Example 28

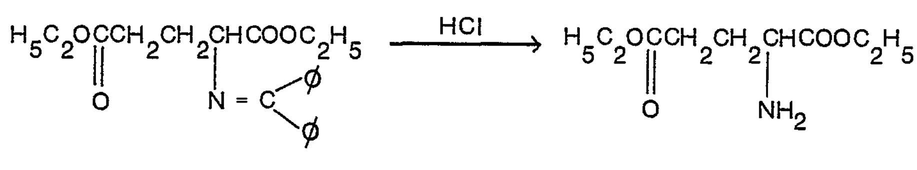


6.6 g (0.018 mol) of N-(diphenylmethylene)-1,3-bis(ethoxycarbonyl)propylamine, 20 ml of ethanol and 3.7 g (0.036 mol) of 35wt% aqueous hydrochloric acid solution were introduced into an Erlenmyer flask and stirred at room temperature for 1 hour.

Then, ethanol was evaporated in a reduced pressure at 15 to 20°C. After 10 ml of benzene was added to the concentrate, the mixture was extracted with water. The water layer was distilled off under a reduced pressure.

Thus, 3.9 g (91.5%) of crude diethylglutamate hydrochloride was obtained.

Example 29

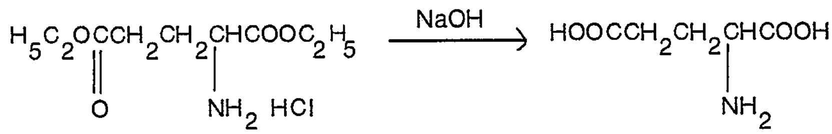


A mixture of 2.4 g (0.01 mol) of diethyl glutamate hydrochloride and 50 ml (0.05 mol) of 1 N aqueous sodium hydroxide solution were introduced into an Erlenmyer flask and stirred at 60°C for 1 hour.

Then, the resulting aqueous solution was passed through an anion-exchange resin (PA 316 made by Mitsubishi Chemical Ind. Ltd., Japan) to give 1.3 g (85.9%) of glutmic acid.

Example 30

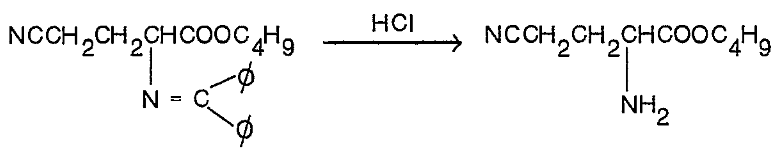


A mixture of 3.5 g (0.01 mol) of N-(diphenylmethylene)-1-butoxycarbonyl-3-cyanopropylamine, 30 ml of cyclohexane, and 20 ml of 1 N aqueous hydrochloric acid solution were introduced into an Erlenmyer flask and stirred at room temperature for 3 hours.

Then, the reaction mixture was treated in a similar manner as Example 25, and 1.7 g (92.4%) of 1-butoxycarbonyl-3-cyanopropylamine were obtained.

Example 31

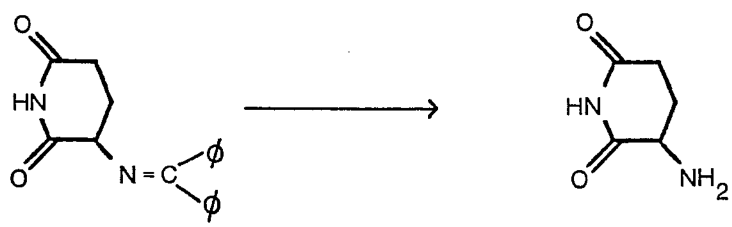

A mixture of 2.9 g (0.01 mol) of N-(diphenylmethylene)-1,3-dicarboxyiminopropylamine and 30 ml of ethanol were introduced into an Erlenmyer flask and stirred at 0 to 5°C.

A solution of 0.9 g of 35 wt% aqueous hydrochloric acid solution and 20 ml of ethanol was added slowly and stirred at 0 to 5°C for 2 hours. The precipitate was filtered and washed with 20 ml of anhydrous ethanol, and obtained 1.4 g (88.0%) of crude 1,3-dicarboxyiminopropylamine hydrochloride.

The crude product was recrystallized from ethanol, affording 1.3 g of pure 1,3-dicarboxyiminopropylamine hydrochloride.

This compound decomposed at 300 to 301°C.

Further, the ethanol layer was distilled off under a reduced pressure, affording 1.7 g (95.1%) of benzophenone.

Example 32

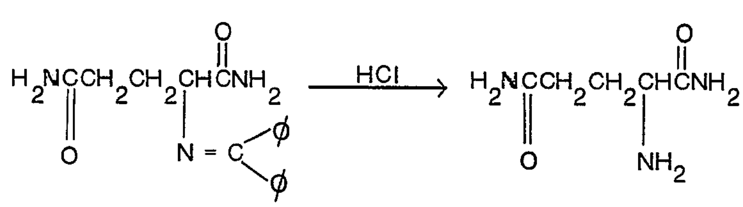


A mixture of 3.1 g (0.01 mol) of N-(diphenylmethylene)-1,3-dicarbamoyl, 30 ml of ethanol, and 20 ml of 1 N aqueous hydrochloric acid solution were introduced into an Erlenmyer flask and stirred at room temperature for 1 hour.

Then, the reaction mixture was treated in a similar manner as Example 25, and obtained 1.3 g (90.0%) of 1,3-dicarbamoylpropylamine.

Further, the benzene layer was distilled under reduced pressure, affording 1.7 g (93.4%) of benzophenone.

Example 33

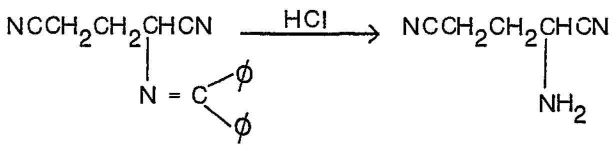


A mixture of 2.7 g (0.01 mol) of N-(diphenylmethylene)-1,3-dicyanopropylamine, 30 ml of ethanol and 12 ml of 1 N aqueous hydrochloric acid solution were introduced into an Erlenmyer flask and stirred at room temperature for 1 hour.

Then, the reaction mixture was treated in a similar manner as Example 25, and obtained 1.3 g (91.3%) of 1,3-dicyanopropylamine.

Further, the benzene layer was distilled under reduced pressure, affording 1.8 g (98.9%) of benzophenone.

TABLE 2

| Example No. | *1 R[1] | *2 R[2] | Yield (%) | melting point (°C) | elemental analysis calc. found | | |
|---|---|---|---|---|---|---|---|
| | | | | | O % | H % | N % |
| 1 | 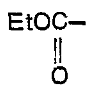 | 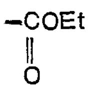 | 89.2 | | 71.91<br>71.64 | 6.86<br>6.82 | 3.81<br>3.65 |
| 2 | 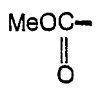 | 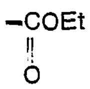 | 88.7 | | 71.37<br>71.33 | 6.56<br>6.78 | 3.96<br>3.97 |
| 3 | 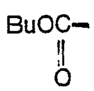 | 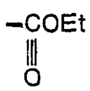 | 90.1 | | 72.86<br>72.96 | 7.39<br>7.24 | 3.54<br>3.46 |
| 4 | 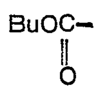 | 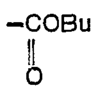 | 90.6 | | 73.73<br>73.59 | 7.85<br>7.61 | 3.31<br>3.57 |
| 5 | 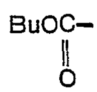 | 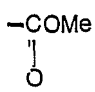 | 87.4 | | 72.42<br>72.23 | 7.13<br>7.19 | 3.67<br>3.58 |
| 6 | NC– | 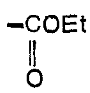 | 90.5 | | 74.98<br>74.92 | 6.29<br>6.31 | 8.74<br>8.56 |
| 7 | NC– | 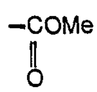 | 89.6 | | 74.49<br>74.20 | 5.92<br>6.11 | 9.14<br>9.18 |

TABLE 2 (Continued)

| Example No. | Infra Red Spectra ($cm^{-1}$) (NaCl) | $^1H$ Nuclear Magnetic Resonance δ (ppm) ($CDCl_3$) | $^{13}C$ Nuclear Magnetic Resonance δ (ppm) ($CDCl_3$) |
|---|---|---|---|
| 1 | 2990, 1740, 1630, 1450, 1380, 1320, 1290, 1250, 1180, 1100, 1030 | 1.1 ~ 1.4(6H), 2.3(4H) 3.9 ~ 4.3(5H), 7.1 ~ 7.7 (10H) | 14.2(2C), 28.6, 30.6, 60.2, 60.8, 64.2, 127 ~ 130(10C) 136.2, 139.3, 170.9, 171.4, 172.7 |
| 2 | 3000, 2960, 1740, 1630, 1450, 1300, 1260, 1200, 1180 | 1.1 ~1.4(3H), 2.2~2.4 (4H), 3.6(3H), 3.9~4.4 (3H), 7.1~7.8(10H) | 14.2, 28.6, 30.3, 51.3, 60.8, 64.1, 127~130(10C), 136.2, 139.2, 170.8, 171.3, 173.0 |
| 3 | 2970, 2880, 1740, 1630, 1450, 1320, 1290, 1250, 1180, 1070, 1030 | 0.7~1.1(3H), 1.1~1.4 (3H), 2.2~2.4(4H), 3.8~4.4(5H), 7.1~7.7 (10H) | 13.7, 14.2, 19.1, 28.7, 30.6, 60.7, 64.1, 64.2, 127~130 (10C), 136.2, 139.3 170.8, 171.3, 172.7 |
| 4 | 2970, 1740, 1630, 1600, 1580, 1495, 1470, 1450, 1390, 1320, 1160~1300 (broad) | 0.8 ~ 1.1(6H) 1.1 ~ 1.8(8H) 2.2~ 2.4(4H) 3.9 ~ 4.3(5H), 7.1 ~ 7.7 (10H) | |
| 5 | 2970, 1740, 1630, 1450, 1320, 1260, 1200, 1170 | 0.7~1.1(3H), 1.1~1.7 (4H), 2.2~2.5(4H), 3.6(3H), 3.8~4.3(3H) 7.0~7.8(10H) | 13.7, 19.1, 28.7, 30.6, 52.1, 64.2(2C) 127~130(10C), 136.1, 139.2, 171.0, 172.0, 172.8 |
| 6 | 3000, 2950, 2250, 1740, 1630, 1600, 1580, 1450, 1320, 1300, 1260, 1210, 1180, 1100, 1030 | 1.1~1.4(3H) 2.2~2.6(4H) 3.9~4.3(3H) 7.1~7.8(10H) | 13.7, 14.1, 29.3, 61.3, 63.1, 119.2, 127~131 (10C), 135.8, 138.9, 170.6, 172.2 |
| 7 | 2960, 2250, 1740, 1630, 1450, 1300, 1210, 1180 | 2.0 ~2.6(4H) 3.6(3H) 4.0 ~4.3(1H) 7.0~7.8(10H) | 13.7, 29.3, 52.2, 63.0, 119.1, 127~131(10C) 135.7, 138.9, 171.1, 172.2 |

TABLE 2 (Continued)

| Example No. | *1 $R^1$ | *2 $R^2$ | Yield (%) | melting point (°C) | elemental analysis calc. found | | |
|---|---|---|---|---|---|---|---|
| | | | | | O % | H % | N % |
| 8 | EtOC–<br>‖<br>O | –CN | 95.3 | | 74.98<br>74.79 | 6.29<br>6.25 | 8.74<br>8.81 |
| 9 | MeOC–<br>‖<br>O | –CN | 91.4 | | 74.49<br>74.19 | 5.92<br>6.13 | 9.14<br>8.85 |
| 10 | BuOC–<br>‖<br>O | –CN | 90.9 | | 75.83<br>75.80 | 6.94<br>6.81 | 8.04<br>7.69 |
| 11 | NC– | –CN | 87.9 | 83 ∼ 4 | 79.10<br>79.01 | 5.53<br>5.64 | 15.37<br>15.27 |
| 12 | $H_2$NC–<br>‖<br>O | –CN | 91.4 | 131 ∼ 2 | 74.20<br>73.99 | 5.88<br>5.76 | 14.42<br>14.32 |
| 13 | $H_2$NC–<br>‖<br>O | –CN$H_2$<br>‖<br>O | 77.4 | 217 ∼ 9 | 69.88<br>69.92 | 6.19<br>6.11 | 13.58<br>13.47 |
| 14 | NO– | –CN$H_2$<br>‖<br>O | 88.6 | 141 ∼ 2 | 74.20<br>74.16 | 5.88<br>5.79 | 14.42<br>14.51 |

TABLE 2 (Continued)

| Example No. | Infra Red Spectra ($cm^{-1}$) (NaCl) | $^{1}H$ Nuclear Magnetic Resonance δ (ppm) ($CDCl_3$) | $^{13}C$ Nuclear Magnetic Resonance δ (ppm) ($CDCl_3$) |
|---|---|---|---|
| 8 | 3000, 2950, 2250, 1740, 1620, 1600, 1580, 1450, 1380, 1320, 1300, 1260, 1180 | 1.1~1.4(3H) 2.1~2.7(4H) 3.9~4.5(3H) 7.1~7.8(10H) | 14.1, 29.9(2C), 51.9, 60.5, 118.9, 127~131 (10C), 135.0, 138.2, 171.8, 173.5 |
| 9 | 2950, 2250, 1740, 1620, 1600, 1580, 1450, 1380, 1320, 1300, 1250, 1200, 1180 | 2.1~2.7(4H) 3.6(3H) 4.2~4.5(1H) 7.1~7.7(10H) | 29.7, 29.9, 51.7, 51.8, 118.9, 127~131(10C) 135.0, 138.2, 172.4, 173.6 |
| 10 | 2970, 2250, 1740, 1620, 1450, 1320, 1300, 1260, 1180 | 0.7~1.1(3H) 1.1~1.8(4H) 2.1~2.7(4H), 3.9~4.5(3H), 7.1~7.8(10H) | 13.6, 19.0, 29.9(2C) 30.5, 51.8, 64.4, 118.9. 127~131(10C), 135.0, 138.2, 171.8, 173.4 |
| 11 | 3040, 2970, 2950, 2250, 1620, 1600, 1580, 1495, 1450, 1430, 1330, 1300, 1290, 1060 | 2.1~2.8(4H) 4.2~4.5(1H) 7.1~7.8(10H) | 13.5, 30.6, 50.9, 118.0 (2C), 127~132(10C) 134.7, 137.8, 175.0 |
| 12 | 3450, 3150, 2250, 1700, 1620, 1450, 1420, 1310, 1300, 1180 | 2.1~2.6(4H) 4.2~4.5(1H) 5.3~5.8(2H) 7.0~7.7(10H) | |
| 13 | 3450, 3370, 3200, 1670, 1630, 1450, 1320, 1290 | 1.8~2.0(4H) 3.7~3.9(1H) 6.8~7.6(10H) ($D_2O$) | |
| 14 | 3480, 3400~3200, (broad) 3060, 2250, 1680, 1620, 1600, 1580, 1450, 1320, 1280 | 1.9~2.6(4H) 3.9~4.2(1H) 6.3~6.7(2H) 7.0~7.8(10H) | |

TABLE 2 (Continued)

| Example No. | *1 $R^1$ | *2 $R^2$ | Yield (%) | melting point (°C) | elemental analysis calc. found C % | H % | N % |
|---|---|---|---|---|---|---|---|
| 15 | 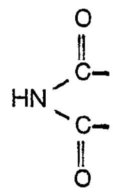 | | 90.8 | 128–9 | 73.94 | 5.52 | 9.58 |
| | | | | | 74.10 | 5.74 | 9.40 |

| Example No. | Infra Red Spectra ($cm^{-1}$) (NaCl) | $^1H$ Nuclear Magnetic Resonance $\delta$ (ppm) ($CDCl_3$) | $^{13}C$ Nuclear Magnetic Resonance $\delta$ (ppm) ($CDCl_3$) |
|---|---|---|---|
| 15 | 3200, 3100, 2850, 1720, 1630, 1360, 1260, 1200 | 1.8 – 2.3(2H), 2.4 – 3.1(2H), 4.0 – 4.3(1H), 7.0 – 7.7(10H), 7.8 – 8.1(1H) | 26(1C), 29(1C), 62(1C), 128 – 139(12C), 171(1C) 172(2C) |

*1, *2: In table 2, $R^1$ and $R^2$ individually represent a radical in a general formula of

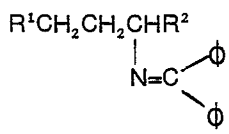

## Claims

1. A propylamine derivative represented by the general formula (I)

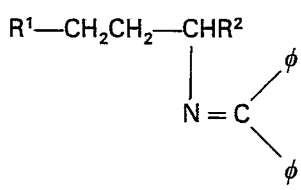

(I)

wherein $R^1$ and $R^2$ are identical or different radicals selected from the group consisting of methoxycarbonyl, ethoxycarbonyl, propoxycarbonyl, butoxycarbonyl, cyano and carbamoyl, or $R^1$ and $R^2$ together form the group —CO—NH—CO, and $\phi$ is a phenyl radical.

2. A compound as claimed in claim 1, characterized in that one of $R^1$ and $R^2$ is a radical selected from the group consisting of methoxycarbonyl, ethoxycarbonyl, propoxycarbonyl, butoxycarbonyl, and the other of $R^1$ and $R^2$ is not carbamoyl.

3. A process of manufacturing a propylamine derivative represented by the general formula (I)

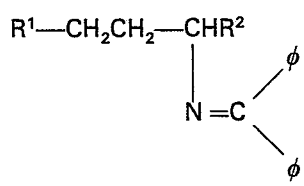

(I)

wherein $R^1$ is a radical selected from the group consisting of methoxycarbonyl, ethoxycarbonyl, propoxycarbonyl, butoxycarbonyl, cyano and carbamoyl, and $R^2$ is a radical selected from the group consisting of methoxycarbonyl, ethoxycarbonyl, propoxycarbonyl, butoxycarbonyl or cyano, and $\phi$ is a phenyl radical, said process being characterized by subjecting olefins represented by the general formula (II)

$$R^1—CH = CH_2 \qquad (II)$$

(wherein $R^1$ is a radical selected from the group consisting of methoxycarbonyl, ethoxycarbonyl, propoxycarbonyl, butoxycarbonyl, cyano, and carbamoyl), to reaction with Schiff bases represented by the general formula (III)

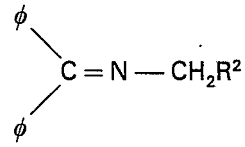

(III)

(wherein $R^2$ is a radical selected from the group consisting of methoxycarbonyl, ethoxycarbonyl, propoxycarbonyl, butoxycarbonyl, cyano and $\phi$ is a phenyl radical), at a reaction temperature of from −80°C to 80°C, with or without use of an ammonium compound as a catalyst in the presence of a base in a monophase or two-phase solvent system.

4. A process of manufacturing a propylamine derivative represented by the general formula (I)

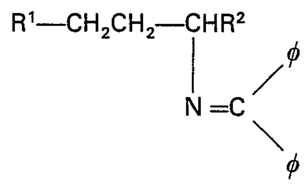

(I)

wherein $R^1$ and $R^2$ together form the group —CO—NH—CO—, and $\phi$ is a pheny radical, siad process being characterized by subjecting olefins represented by the general formula (II)

$$R^1—CH = CH_2 \qquad (II)$$

(wherein $R^1$ is a carbamoyl radical, to reaction with Schiff bases represented by the general formula (III)

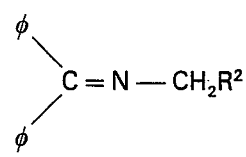

(III)

(wherein $R^2$ is a radical selected from the group consisting of methoxycarbonyl, ethoxycarbonyl, propoxycarbonyl, butoxycarbonyl, cyano and $\phi$ is a phenyl radical), at a temperature of from −80°C to 80°C, in the presence of a base in a monophase organic solvent system.

5. A process as claimed in claim 3, characterized in that the catalyst is selected from the group consisting of tetraalkylammonium halogenide, tetraalkylammonium hydrogensulfate, tetraalkylammonium hydroxide, benzyltrialkylammonium halogenide, benzyltrialkyl hydrogensulfate, and benzyltrialkylammonium hydroxide.

6. A process as claimed in claim 3, characterized in that the catalyst is employed in an amount of from 0.0001 to 1 equivalent, based on the Schiff base.

7. A process as claimed in claim 3 or claim 4, characterized in that the reaction temperature is in the range of from −80°C to 40°C.

8. A process as claimed in claim 3 or claim 5, characterized in that the base is employed in an amount of 0.001 to 10 equivalents, based on the Schiff base.

9. A process as claimed in claim 3 or claim 5, characterized in that the monophase solvent system is a solvent selected from the group consisting of aliphatic alcohols such as methanol, ethanol, propanol, butanol; halogenated hydrocarbons such as dichloromethane, chloroform, trichloroethylene, trichloroethane; aromatic hydrocarbons such as benzene, toluene, xylene; ethers such as diethyl ether, tetrahydrofuran, dioxane; dimethylformamide, and dimethylsulfoxide.

10. A process as claimed in claim 9, characterized in that the base is alkali metal hydroxides, alkali metals, alkali metal hydrides, alkali metral amides or alcoholates of alkali metals.

11. A process as claimed in claim 3, characterized in that the two-phase solvent system comprises (a) water and (b) at least one kind of organic solvent selected from the group consisting of halogenated hydrocarbons such as dichloromethane, chloroform, trichloroethylene, trichloroethane; aromatic hydrocarbons such as benzene, toluene, xylene; ethers such as diethylether, tetrahydrofuran, dioxane, dimethylformamide, and dimethylsulfoxide.

12. A process as claimed in claim 11, characterized in that the base is at least one kind selected from the group consisting of hydroxides, carbonates and hydrogencarbonates of alkalimetals.

13. A process of manufacturing a propylamine derivative represented by the general formula (I)

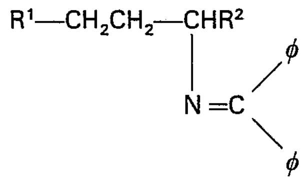

(I)

(wherein $R^1$ and $R^2$ are identical or different radicals selected from the group consisting of cyano, and carbamoyl, with at least one of $R^1$ and $R^2$ being carbamoyl), said process being characterized by subjecting a propylamine derivative represented by the general formula (I)

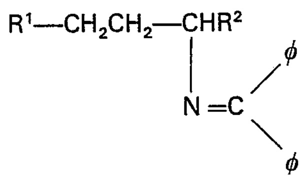

(I)

wherein $R^1$ is a radical selected from the group consisting of methoxycarbonyl, ethoxycarbonyl, propoxycarbonyl, butoxycarbonyl, cyano, carbamoyl, and $R^2$ is a radical selected from the group consisting of methoxycarbonyl, ethoxycarbonyl, propoxycarbonyl, butoxycarbonyl, cyano, or $R^1$ and $R^2$ together form the group —CO—NH—CO, to reaction with ammonia at a temperature of from −20°C to 80°C under atmospheric pressure or under increased or reduced pressure in the presence of a solvent, whereby methoxycarbonyl, ethoxycarbonyl, propoxycarbonyl or butoxycarbonyl radical is converted to carbamoyl radical and the group —CO—NH—CO— is converted to two carbamoyl radicals.

14. A process of manufacturing a propylamine derivative represented by the general formula (I)

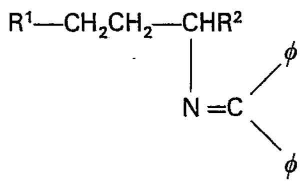

(I)

wherein $R^1$ and $R^2$ together form the group —CO—NH—CO—, and $\phi$ is a phenyl radical, characterized by subjecting a propylamine derivative represented by the general formula (I)

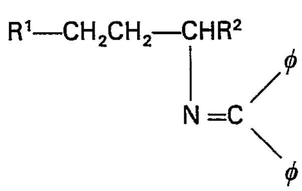

(I)

(wherein $R^1$ and $R^2$ are identical or different radicals selected from the group consisting of methoxycarbonyl, ethoxycarbonyl, propoxycarbonyl, and butoxycarbonyl, and $\phi$ is a phenyl radical), to reaction with 0.8 to 1.1 moles of ammonia, based on the starting material, at a temperatures of from −20 to 80°C under atmospheric pressure or under increased or reduced pressure in an organic solvent.

15. A process as claimed in claim 13 or 14, characterized in that the solvent is selected from the group consisting of water; aliphatic alcohols such as methanol, ethanol, propanol, butanol; halogenated hydrocarbons such as dichloromethane, chloroform, trichloroethylene; aromatic hydrocarbons such as benzene, toluene, xylene; ethers such as diethyl ether, tetrahydrofuran, dioxane; dimethylformamide, and dimethylsulfoxide.

16. A process as claimed in claim 13 or claim 14, characterized in that the reaction temperature is in the range of from 0 to 40°C, and the reaction pressure is atmospheric pressure.

**Revendications**

1. Dérivé de propylamine représenté par la formule générale (I)

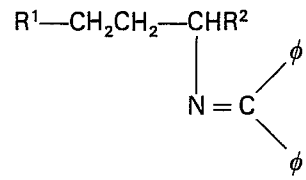
(I)

où $R^1$ et $R^2$ sont des radicaux identiques ou différents, choisis dans le groupe se composant de groupes méthoxycarbonyle, éthoxycarbonyle, propoxycarbonyle, butoxycarbonyle, cyano et carbamoyle, ou bien $R^1$ et $R^2$ forment ensemble le groupe —CO—NH—CO—, et $\phi$ est un radical phényle.

2. Composé selon la revendication 1, caractérisé en ce qu'un des $R^1$ et $R^2$ est un radical choisi dans le groupe se composant de groupes méthoxycarbonyle, éthoxycarbonyle, propoxycarbonyle, butoxycarbonyle et l'autre des $R^1$ et $R^2$ n'est pas le groupe carbamoyle.

3. Procédé de fabrication d'un dérivé de propylamine représenté par la formule générale (I)

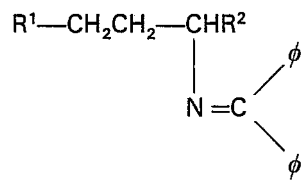
(I)

où $R^1$ est un radical choisi dans le group se composant de groupes méthoxycarbonyle, éthoxycarbonyle, propoxycarbonyle, butoxycarbonyle, cyano et carbamoyle, et $R^2$ est un radical choisi dans le groupe se composant de groupes méthoxycarbonyle, éthoxycarbonyle, propoxycarbonyle, butoxycarbonyle ou cyano, et $\phi$ est un radical phényle, ce procédé étant caractérisé en ce qu'on soumet des oléfines représentées par la formule générale (II)

$$R^1—CH = CH_2 \quad (II)$$

(où $R^1$ est un radical choisi dans le groupe se composant de radicaux méthoxycarbonyle, éthoxycarbonyle, propoxycarbonyle, butoxycarbonyle, cyano et carbamoyle), à une réaction avec des bases de Schiff représentées par la formule générale (III)

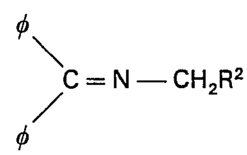
(III)

(où $R^2$ est un radical choisi dans le groupe se composant de groupes méthoxycarbonyle, éthoxycarbonyle, propoxycarbonyle, butoxycarbonyle, cyano et $\phi$ est un radical phényle), à une température de réaction de −80°C à 80°C, avec ou sans utilisation d'un composé d'ammonium comme catalyseur, en présence d'une base, dans un système de solvants monophasé ou biphasé.

4. Procédé de fabrication d'un dérivé de propylamine représenté par la formule générale (I)

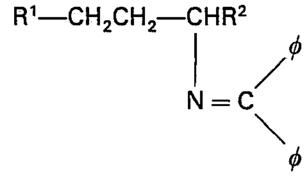
(I)

où $R^1$ et $R^2$ ensemble forment le groupe —CO—NH—CO—, et $\phi$ est un radical phényle, ce procédé étant caractérisé en ce qu'on soumet des oléfins représentées par la formule générale (II)

$$R^1—CH = CH_2 \quad (II)$$

où $R^1$ est un radical carbamoyle, à une réaction avec une base de Schiff représentée par la formule générale (III)

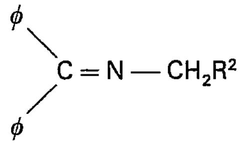

(III)

(où $R^2$ est un radical choisi dans le groupe se composant de groupes méthoxycarbonyle, éthoxycarbonyle, propoxycarbonyle, butoxycarbonyle et $\phi$ est un radical phényle), à une température de $-80°C$ à $80°C$, en présence d'une base, dans un système de solvants organique monophasé.

5. Procédé selon la revendication 3, caractérisé en ce que le catalyseur est choisi dans le groupe se composant d'halogénure de tétraalkylammonium, de sulfate acide de tétraalkylammonium, d'hydroxyde de tétraalkylammonium, d'halogénure de benzyltrialkylammonium, de sulfate acide de benzyltrialkylammonium et d'hydroxyde de benzyltrialkylammonium.

6. Procédé selon la revendication 3, caractérisé en ce que le catalyseur est employé en quantité de 0,0001 à l'équivalent, en se basant sur la base de Schiff.

7. Procédé selon la revendication 3 ou la revendication 4, caractérisé en ce que la température de réaction est dans l'intervalle de $-80°C$ à $40°C$.

8. Procédé selon la revendication 3 ou la revendication 5, caractérisé en ce que la base est employée en quantité de 0,001 à 10 équivalents, en se basant sur la base de Schiff.

9. Procédé selon la revendication 3 ou la revendication 5, caractérisé en ce que le système de solvants monophasé est un solvant choisi dans le groupe se composant d'alcools aliphatiques, tels que le méthanol, l'éthanol, le propanol, le butanol, d'hydrocarbures halogénés tels que le dichlorométhane, le chloroforme, le trichloroéthylène, le trichloroéthane, d'hydrocarbures aromatiques tels que le benzène, le toluène, le xylène, d'éthers tels que l'éther diéthylique, le tétrahydrofurane, le dioxane, de la diméthylformamide et du diméthylsulfoxyde.

10. Procédé selon la revendication 9, caractérisé en ce que la base est formée d'hydroxydes de métaux alcalins, de métaux alcalins, d'hydrures de métaux alcalins, d'amidures de métaux alcalins ou d'alcoolates de métaux alcalins.

11. Procédé selon la revendication 3, caractérisé en ce que le système de solvants biphasé comprend (a) de l'eau et (b) au moins un genre de solvant organique choisi dans le groupe se composant d'hydrocarbures halogénés, tels que le dichlorométhane, le chloroforme, le trichloroéthylène, le trichloroéthane; d'hydrocarbures aromatiques tels que le benzène, le toluène, le xylène, d'éthers tels que l'éther diéthylique, le tétrahydrofurane, le dioxane, de la diméthylformamide et du diméthylsulfoxyde.

12. Procédé selon la revendication 11, caractérisé en ce que la base est au moins un genre choisi dans le groupe se composant d'hydroxydes, de carbonates et de bicarbonates de métaux alcalins.

13. Procédé de fabrication d'un dérivé de propylamine représenté par la formule générale (I)

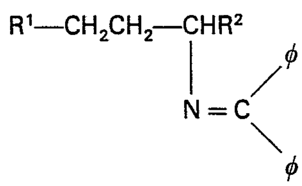

(I)

(où $R^1$ et $R^2$ sont des radiaux identiques ou différents, choisis dans le groupe se composant de groupes cyano et carbamoyle, au moins un des $R^1$ et $R^2$ étant le groupe carbamoyle), ce procédé étant caractérisé en ce qu'on soumet un dérivé de propylamine représenté par la formule générale (I)

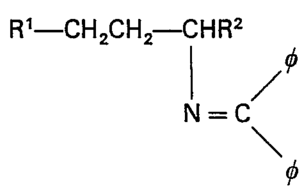

(I)

où $R^1$ est un radical choisi dans le groupe se composant de groupes méthoxycarbonyle, éthoxycarbonyle, propoxycarbonyle, butoxycarbonyle, cyano, carbamoyle, et $R^2$ est un radical choisi dans le groupe se composant de groupes méthoxycarbonyle, éthoxycarbonyle, propoxycarbonyle, butoxycarbonyle, cyano, ou bien $R^1$ et $R^2$ forment ensemble le groupe —CO—NH—CO— à la réaction avec l'ammoniaque à une température de $-20°C$ à $80°C$, sous la pression atmosphérique, ou sous pression augmentée ou réduite, en présence d'un solvant, et, de se fait, le radical méthoxycarbonyle, éthoxycarbonyle, propoxycarbonyle, ou butoxycarbonyle est transformé en radical carbamoyle et le groupe —CO—NH—CO est transformé en deux radicaux carbamoyle.

14. Procédé de fabrication d'un dérivé de propylamine représenté par la formule générale (I)

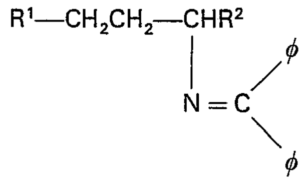
(I)

où $R^1$ et $R^2$ ensemble forment le groupe —CO—NH—CO—, et $\phi$ est un radical phényle, caractérisé en ce qu'on soumet un dérivé de propylamine représenté par la formule générale (I)

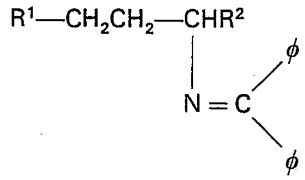
(I)

(où $R^1$ et $R^2$ sont des radicaux identiques ou différents, choisis dans le groupe se composant de groupes méthoxycarbonyle, éthoxycarbonyle, propoxycarbonyle et butoxycarbonyle, et $\phi$ est un radical phényle) à la réaction avec 0,8 à 1,1 mole d'ammoniac, en se basant sur la matière de départ, à une température de —20 à 80°C sous la pression atmosphérique ou sous pression augmentée ou réduite, dans un solvant organique.

15. Procédé selon la revendication 13 ou la revendication 14, caractérisé en ce que le solvant est choisi dans le groupe se composant d'eau; d'alcools aliphatiques tels que le méthanol, l'éthanol, le propanol, le butanol; d'hydrocarbures halogénés tels que le dichlorométhane, le chloroforme, le trichloroéthylène, d'hydrocarbures aromatiques tels que le benzène, le toluène, le xylène; d'éthers tels que l'éther diéthylique, le tétrahydrofurane, li dioxane, de la diméthylformamide et du diméthylsulfoxyde.

16. Procédé selon la revendication 13 ou la revendication 14, caractérisé en ce que la température de réaction est dans l'intervalle de 0 à 40°C et la pression de réaction est la pression atmosphérique.

**Patentansprüche**

1. Propylaminderivat der allgemeinen Formel (I)

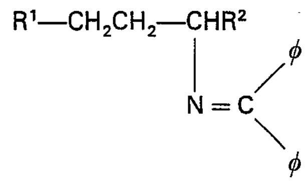
(I)

in der $R^1$ und $R^2$ gleich oder unterschiedlich und aus der aus Methoxycarbonyl, Äthoxycarbonyl, Propoxycarbonyl, Butoxycarbonyl, Cyano und Carbamyl bestehenden Gruppe ausgewählt sind oder wobei $R^1$ und $R^2$ gemeinsam die —CO—NH—CO-Gruppe bilden, und in der $\phi$ ein Phenylrest ist.

2. Verbindung nach Anspruch 1, dadurch gekennzeichnet, daß einer der Reste $R^1$ und $R^2$ ein aus der aus Methoxycarbonyl, Äthoxycarbonyl, Propoxycarbonyl, Butoxycarbonyl bestehenden Gruppe ausgewählter Rest und der andere der Reste $R^1$ und $R^2$ nicht Carbamyl ist.

3. Verfahren zur Herstellung eines Propylaminderivats drder allgemeinen Formel (I)

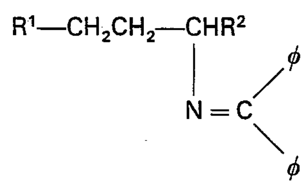
(I)

in der $R^1$ ein aus der aus Methoxycarbonyl, Äthoxycarbonyl, Propoxycarbonyl, Butoxycarbonyl, Cyano und Carbamyl bestehenden Gruppe ausgewählter Rest und $R^2$ ein aus der aus Methoxycarbonyl, Äthoxycarbonyl, Propoxycarbonyl, Butoxycarbonyl oder Cyano bestehenden Gruppe ausgewählter Rest und $\phi$ ein Phenylrest ist, dadurch gekennzeichnet, daß man Olefine der allgemeinen Formel (II)

$R^1—CH—CH_2$ (II),

(in der $R^1$ ein aus der aus Methoxycarbonyl, Äthoxycarbonyl, Propoxycarbonyl, Butoxycarbonyl, Cyano und Carbamyl bestehenden Gruppe ausgewählter Rest ist) mit Schiffschen Basen der allgemeinen Formel (III)

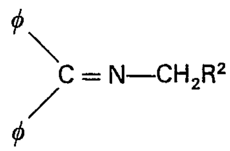 (III)

(wobei $R^2$ ein aus der aus Methoxycarbonyl, Äthoxycarbonyl, Propoxycarbonyl, Butoxycarbonyl, Cyano bestehenden Gruppe ausgewählter Rest und $\phi$ ein Phenylrest ist) bei einer Reaktionstemperatur von —80°C bis 80°C mit oder ohne Anwendung einer Ammoniumverbindung als Katalysator in Gegenwart einer Base in einem einphasigen oder zweiphasigen Lösungsmittelsystem umsetzt.

4. Verfahren zur Herstellung eines Propylaminderivats der allgemeinen Formel (I)

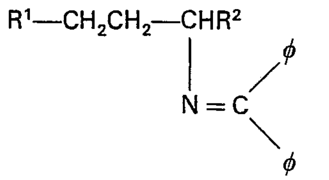 (I),

in der $R^1$ und $R^2$ gemeinsam die —CO—NH—CO-Gruppe bilden und $\phi$ ein Phenylrest ist, dadurch gekennzeichnet, daß man Olefine der allgemeinen Formel (II)

$R^1—CH = CH_2$ (II),

in der $R^1$ eine Carbamylgruppe ist, mit Schiffschen Basen der allgemeinen Formel (III)

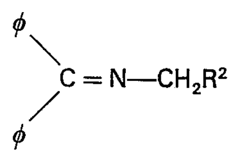 (III)

(wobei $R^2$ ein aus der aus Methoxycarbonyl, Äthoxycarbonyl, Propoxycarbonyl, Butoxycarbonyl bestehenden Gruppe ausgewählter Rest und $\phi$ ein Phenylrest ist) bei einer Temperatur zwischen —80°C und 80°C in Gegenwart einer Base in einem einphasigen organischen Lösungsmittelsystem umsetzt.

5. Verfahren nach Anspruch 3, dadurch gekennzeichnet, daß der Katalysator aus der aus Tetraalkylammoniumhalogenid, Tetraalkylammoniumhydrogensulfat, Tetraalkylammoniumhydroxid, Benzyltrialkylammoniumhalogenid, Benzyltrialkylhydrogensulfat und Benzyltrialkylammoniumhydroxid bestehenden Gruppe ausgewählt wird.

6. Verfahren nach Anspruch 3, dadurch gekennzeichnet, daß der Katalysator in einer Menge von 0,0001 bis 1, Äquivalent, bezogen auf die Schiffsche Base, eingesetzt wird.

7. Verfahren nach Anspruch 3 oder 4, dadurch gekennzeichnet, daß die Reaktionstemperatur im Bereich von —80°C bis 40°C liegt.

8. Verfahren nach Anspruch 3 oder 5, dadurch gekennzecichnet, daß die Base in einer Menge von 0,0001 bis 10 Äquivalent, bezogen auf die Schiffsche Base, eingesetzt wird.

9. Verfahren nach Anspruch 3 oder 5, dadurch gekennzeichnet, daß das einphasige Lösungsmittelsystem ein aus der Gruppe der aliphatischen Alkohole, wie Methanol, Äthanol, Propanol, Butanol; halogenierten Kohlenwasserstoffe, wie Dichlormethan, Chloroform, Trichloräthylen, Trichloräthan; aromatischen Kohlenwasserstoffe, wie Benzol, Toluol, Xylol; Äthern, wie Diäthyläther, Tetrahydrofuran, Dioxan; Dimethylformamid und Dimethylsulfoxid ausgewähltes Lösungsmittel ist.

10. Verfahren nach Anspruch 9, dadurch gekennzeichnet, daß die Base ein Alkalimetallhydroxid, Alkalimetall, Alkalimetallhydrid, Alkalimetallamid oder ein Alkoholat von Alkalimetallen ist.

11. Verfahren nach Anspruch 3, dadurch gekennzeichnet, daß das zweiphasige Lösungsmittelsystem aus (a) Wasser und (b) mindestens einem organischen Lösungsmittel, ausgewählt aus der aus halogenierten Kohlenwasserstoffen, wie Dichlormethan, Chloroform, Trichloräthylen, Trichloräthan; aromatischen Kohlenwasserstoffen, wie Benzol, Toluol, Xylol; Äthern, wie Diäthyläther, Tetrahydrofuran, Dioxan; Dimethylformamid und Dimethylsulfoxid bestehenden Gruppe, besteht.

12. Verfahren nach Anspruch 11, dadurch gekennzeichnet, daß die Base mindestens eine der aus

Hydroxiden, Carbonaten und Hydrogencarbonaten von Alkalimetallen bestehenden Gruppe ist.

13. Verfahren zur Herstellung eines Propylamin derivats der allgemeinen Formel (I)

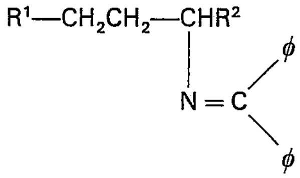
(I)

in der $R^1$ und $R^2$ gleich oder unterschiedlich und aus der aus Cyano und Carbamyl bestehenden Gruppe ausgewählt sind, wobei mindestens einer der Reste $R^1$ und $R^2$ eine Carbamyl-gruppe ist), dadurch gekennzeichnet, daß man ein Propylaminderivat der allgemeinen Formel (I)

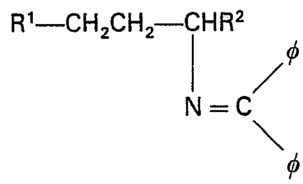
,

in der $R^1$ ein aus der aus Methoxycarbonyl, Äthoxycarbonyl, Propoxycarbonyl, Butoxycarbonyl, Cyano, Carbamyl, bestehenden Gruppe ausgewählter Rest und $R^2$ ein aus der aus Methoxycarbonyl, Äthoxycarbonyl, Propoxycarbonyl, Butoxycarbonyl, Cyano bestehenden Gruppe ausgewählter Rest ist oder $R^1$ und $R^2$ gemeinsam die —CO—NH—CO-Gruppe bilden, mit Ammoniak bei einer Temperatur zwischen —20°C und 80°C unter Atmosphärendruck oder bei erhöhtem oder vermindertem Druck in Gegenwart eins Lösungsmittels umsetzt, wodurch die Methoxycarbonyl-, Äthoxycarbonyl-, Propoxycarbonyl- oder Butoxycarbonylgruppe in eine Carbamylgruppe umgewandelt und die —CO—NH—CO-Gruppe in zwei Carbamylgruppen umgewandelt wird.

14. Verfahren zur Herstellung eines Propylaminderivats der allgemeinen Formel (I)

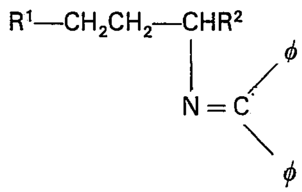
(I),

in der $R^1$ und $R^2$ gemeinsam die —CO—NH—CO-Gruppe bilden und $\phi$ ein Phenylrest ist, dadurch gekennzeichnet, daß man ein Propylaminderivat der allgemeinen Formel (I)

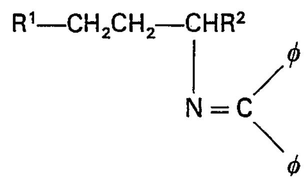
(I),

(wobei $R^1$ und $R^2$ gleich oder unterschiedlich und aus der aus Methoxycarbonyl, Äthoxycarbonyl, Propoxycarbonyl und Butoxycarbonyl bestehenden Gruppe ausgewählte Reste sind und $\phi$ ein Phenylrest ist) mit 0,8 bis 1,1 Molen Ammoniak, bezogen auf das Ausgangsmaterial, bei einer zwischen —20°C und 80°C liegenden Temperatur unter Atmosphärendruck oder unter erhöhtem oder vermindertem Druck in einem organischen Lösungsmittel umsetzt.

15. Verfahren nach Anspruch 13 oder 14, dadurch gekennzeichnet, daß das Lösungsmittel ein aus der Gruppe Wasser; aliphatischen Alkoholen, wie Methanol, Äthanol, Propanol, Butanol; halogenierten Kohlenwasserstoffen, wie Dichlormethan, Chloroform, Trichloräthylen; aromatischen Kohlenwasserstoffen, wie Benzol, Toluol, Xylol; Äthern, wie Diäthyläther, Tetrahydrofuran, Dioxan; Dimethylformamid und Dimethylsulfoxid ausgewähltes Lösungsmittel ist.

16. Verfahren nach Anspruch 13 oder 14, dadurch gekennzeichnet, daß die Reaktionstemperatur im Bereich von 0 bis 40°C liegt und der Reaktionsdruck Amostphärendruck beträgt.